(19) Europäisches Patentamt
European Patent Office
Office européen des brevets

(11) Veröffentlichungsnummer: **0 370 453 B1**

(12) # EUROPÄISCHE PATENTSCHRIFT

(45) Veröffentlichungstag der Patentschrift: **15.06.94**

(51) Int. Cl.[5]: **C07K 99/20**

(21) Anmeldenummer: **89121498.3**

(22) Anmeldetag: **21.11.89**

Die Akte enthält technische Angaben, die nach dem Eingang der Anmeldung eingereicht wurden und die nicht in dieser Patentschrift enthalten sind.

(54) **Peptide mit bradykinin-antagonistischer Wirkung.**

(30) Priorität: **24.11.88 DE 3839581**
**19.05.89 DE 3916291**
**03.06.89 DE 3918225**

(43) Veröffentlichungstag der Anmeldung:
**30.05.90 Patentblatt 90/22**

(45) Bekanntmachung des Hinweises auf die Patenterteilung:
**15.06.94 Patentblatt 94/24**

(84) Benannte Vertragsstaaten:
**AT BE CH DE ES FR GB GR IT LI LU NL SE**

(56) Entgegenhaltungen:
**EP-A- 0 190 058**
**WO-A-86/07263**

(73) Patentinhaber: **HOECHST AKTIENGESELLSCHAFT**

**D-65926 Frankfurt(DE)**

(72) Erfinder: **Henke, Stephan, Dr.**
**Wingertstrasse 2c**
**D-6238 Hofheim am Taunus(DE)**
Erfinder: **Anagnostopulos, Hiristo**
**Feldbergstrasse 6**
**D-6204 Taunusstein(DE)**
Erfinder: **Breipohl, Gerhard, Dr.**
**Geisenheimer Strasse 95**
**D-6000 Frankfurt am Main 71(DE)**
Erfinder: **Knolle, Jochen, Dr.**
**Höchster Strasse 21**
**D-6239 Kriftel(DE)**
Erfinder: **Stechel, Jens, Dr.**
**Loreleistrasse 7**
**D-6230 Frankfurt am Main 80(DE)**
Erfinder: **Schölkens, Bernward, Prof. Dr.**
**Hölderlinstrasse 62**
**D-6233 Kelkheim (Taunus)(DE)**
Erfinder: **Fehlhaber, Hans-Wolfram, Dr.**
**Thomas-Mann-Strasse 5a**
**D-6270 Idstein (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Bradykinin-antagonistische Peptide werden in WO 86/07263 beschrieben, bei denen u. a. L-Pro in Position 7 des Peptidhormons Bradykinin oder anderer Bradykininanaloga durch eine D-Aminosäure, wie D-Phe, D-Thi, D-Pal, CDF, D-Nal, MDY, D-Phg, H-His, D-Trp, D-Tyr, D-hPhe, D-Val, D-Ala, D-His, D-Ile, D-Leu und DOMT ersetzt ist.

Der Erfindung liegt die Aufgabe zugrunde, neue wirksame Peptide mit bradykininantagonistischer Wirkung zu finden.

Diese Aufgabe wird gelöst durch die Peptide der Formel I

A-B-C-E-F-K-(D)-Tic-G-M-F'-I (I),

in welcher bedeuten:

A

$a_1$) Wasserstoff, $(C_1\text{-}C_8)$-Alkyl, $(C_1\text{-}C_8)$-Alkanoyl, $(C_1\text{-}C_8)$-Alkoxycarbonyl oder $(C_1\text{-}C_8)$-Alkylsulfonyl,

in welchen jeweils 1, 2 oder 3 Wasserstoffatome gegebenenfalls durch 1, 2 oder drei gleiche oder verschiedene Reste aus der Reihe Carboxy, Amino, $(C_1\text{-}C_4)$-Alkyl, $(C_1\text{-}C_4)$-Alkylamino, Hydroxy, $(C_1\text{-}C_3)$-Alkoxy, Halogen, Di-$(C_1\text{-}C_4)$-alkylamino, Carbamoyl, Sulfamoyl, $(C_1\text{-}C_4)$-Alkoxycarbonyl, $(C_6\text{-}C_{12})$-Aryl und $(C_6\text{-}C_{12})$-Aryl-$(C_1\text{-}C_5)$-alkyl ersetzt sind,

oder in welchen jeweils 1 Wasserstoffatom gegebenenfalls durch einen Rest aus der Reihe $(C_3\text{-}C_8)$-Cycloalkyl, $(C_1\text{-}C_4)$-Alkylsulfonyl, $(C_1\text{-}C_4)$-Alkylsulfinyl, $(C_6\text{-}C_{12})$-Aryl-$(C_1\text{-}C_4)$-alkylsulfonyl, $(C_6\text{-}C_{12})$-Aryl-$(C_1\text{-}C_4)$-alkylsulfinyl, $(C_6\text{-}C_{12})$-Aryloxy, $(C_3\text{-}C_9)$-Heteroaryl und $(C_3\text{-}C_9)$-Heteroaryloxy

und 1 oder 2 Wasserstoffatome durch 1 oder 2 gleiche oder verschiedene Reste aus der Reihe Carboxy, Amino, $(C_1\text{-}C_4)$-Alkylamino, Hydroxy, $(C_1\text{-}C_4)$-Alkoxy, Halogen, Di-$(C_1\text{-}C_4)$-alkylamino, Carbamoyl, Sulfamoyl, $(C_1\text{-}C_4)$-Alkyloxycarbonyl, $(C_6\text{-}C_{12})$-Aryl und $(C_6\text{-}C_{12})$-Aryl-$(C_1\text{-}C_5)$-alkyl ersetzt sind,

$a_2$) $(C_3\text{-}C_8)$-Cycloalkyl, Carbamoyl,

das gegebenenfalls am Stickstoff durch $(C_1\text{-}C_6)$-Alkyl oder $(C_6\text{-}C_{12})$-Aryl substituiert sein kann,

$(C_6\text{-}C_{12})$-Aryl, $(C_7\text{-}C_{18})$-Aryloyl, $(C_6\text{-}C_{12})$-Arylsulfonyl oder $(C_3\text{-}C_9)$-Heteroaryl oder $(C_3\text{-}C_9)$-Heteroaryloyl,

wobei in den unter $a_1$) und $a_2$) definierten Resten jeweils Heteroaryl, Aryloyl, Arylsulfonyl und Heteroaryloyl gegebenenfalls durch 1, 2, 3 oder 4 verschiedene Reste aus der Reihe Carboxy, Amino, Nitro, $(C_1\text{-}C_4)$-Alkylamino, Hydroxy, $(C_1\text{-}C_4)$-Alkyl, $(C_1\text{-}C_4)$-Alkoxy, Halogen, Cyano, Di-$(C_1\text{-}C_4)$-alkylamino, Carbamoyl, Sulfamoyl und $(C_1\text{-}C_4)$-Alkoxycarbonyl substituiert ist, oder

$a_3$) einen Rest der Formel II,

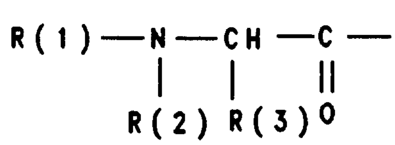

II

mit

R(1) wie A unter $a_1$) oder $a_2$) definiert,

R(2) Wasserstoff oder Methyl,

R(3) Wasserstoff oder $(C_1\text{-}C_6)$-Alkyl, vorzugsweise $(C_1\text{-}C_4)$-Alkyl, das gegebenenfalls durch Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Ntrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert ist,

wobei substituiertes Amino für eine Verbindung -NH-A- und substituiertes Guanidino für eine Verbindung -NH-C(NH)-NH-A stehen, in denen A wie unter $a_1$) oder $a_2$) definiert ist;

B Arg, Lys, Orn, 2,4-Diaminobutyroyl oder einen L-Homoargininrest,

wobei jeweils die Amino- bzw. die Guanidinogruppe der Seitenkette durch A wie unter $a_1$) oder $a_2$) beschrieben substituiert sein kann;

C eine Verbindung der Formel IIIa oder IIIb

G'-G'-Gly (IIIa)

G'-NH-$(CH_2)_n$-CO (IIIb),

mit

G' unabhängig voneinander einem Rest der Formel IV

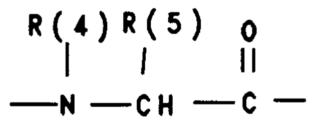

IV

worin R(4) und R(5) zusammen mit den diesen tragenden Atomen ein heterocyclisches mono-, bi- oder tricyclisches Ringsystem mit 2 bis 15 C-Atomen bilden,

und n 2 bis 8 ist;

E den Rest von Phenylalanin,

das gegebenenfalls durch Halogen in der 2-, 3- oder 4-Position substituiert ist,

Tyrosin, O-Methyltyrosin, 2-Thienylalanin, 2-Pyridylalanin oder Naphthylalanin;

F unabhängig voneinander den Rest einer neutralen, sauren oder basischen, aliphatischen oder aromatischen Aminosäure,

die in der Seitenkette substituiert sein kann,

oder eine direkte Bindung;

(D)-Tic den Rest der Formel V

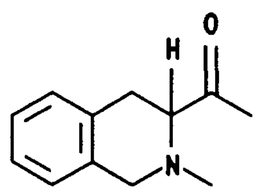

V

G wie G' oder eine direkte Bindung;

F' den Rest der basischen Aminosäuren Arg oder Lys in der L- oder D-Form oder eine direkte Bindung,

wobei die Guanidinogruppe bzw. Aminogruppe der Seitenkette durch A wie unter $a_1$) oder $a_2$) definiert substituiert sein kann,

oder einen Rest -NH-$(CH_2)_n$- mit n gleich 2 - 8,

oder eine direkte Bindung:

I -OH, $-NH_2$ oder $-NHC_2H_5$;

K den Rest -NH-$(CH_2)_x$-CO mit x gleich 1 - 4 oder eine direkte Bindung;

M wie F definiert,

sowie deren physiologisch verträgliche Salze.

Falls nicht anders angegeben, steht die Abkürzung eines Aminosäurerestes ohne einen Stereodeskriptor für den Rest in der L-Form (vgl. Schröder, Lübke, The Peptides, Band I, New York 1965, Seiten XXII-XXIII; Houben-Weyl, Methoden der Organischen Chemie, Band XV/1 und 2, Stuttgart 1974), wie z. B.

Aad, Abu, γAbu, ABz, 2ABz, εAca, Ach, Acp, Adpd, Ahb, Aib, βAib, Ala, βAla, Ala, Alg, All, Ama, Amt, Ape, Apm, Apr, Arg, Asn, Asp, Asu, Aze, Azi, Bai, Bph, Can, Cit, Cys, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, Lys, Met, Mim, Min, nArg, Nle, Nva, Oly, Orn, Pan, Pec, Pen, Phe, Phg, Pic, Pro, Pro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val.

Als Rest eines heterocyclischen Ringsystems der Formel IV kommen insbesondere Reste von Heterocyclen aus der folgenden Gruppe in Betracht:
Pyrrolidin (A); Piperidin (B); Tetrahydroisochinolin (C); Decahydroisochinolin (D); Octahydroindol (E); Octahydrocyclopenta[b]pyrrol (F); 2-Aza-bicyclo[2.2.2]octan (G); 2-Azabicyclo[2.2.1]heptan (H); 2-Azaspiro-[4.5]decan (I); 2-Azaspiro[4.4]nonan (J); Spiro[(bicyclo[2.2.1]heptan)-2,3-pyrrolidin] (K); Spiro[(bicyclo[2.2.2]-octan)-2,3-pyrrolidin] (L); 2-Azatricyclo[4.3.0.1$^{6,9}$]decan (M); Decahydrocyclohepta[b]pyrroi (N); Octahydroisoindol (O); Octahydrocyclopenta[c]pyrrol (P); 2,3,3a,4,5,7a-Hexahydroindol (Q); Tetrahydrothiazol (R); 2-Azablcyclo [3.1.0]hexan (S); Isoxazolidin (T); Pyrazolidin (U); Hydroxyprolin (V); die alle gegebenenfalls substituiert sein können.

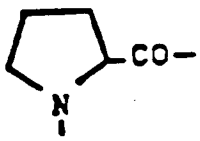
A

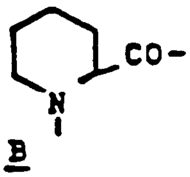
B

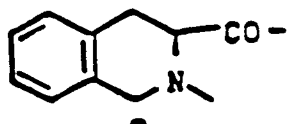
C

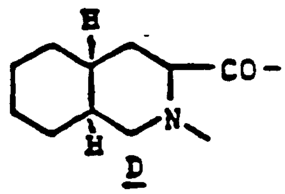
D

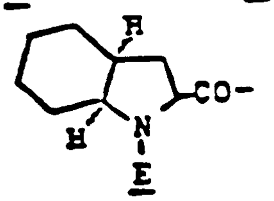
E

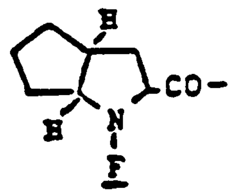
F

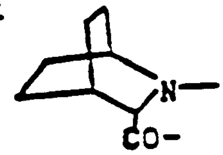
G

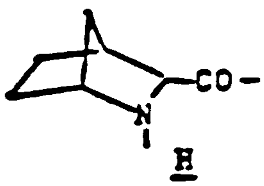
H

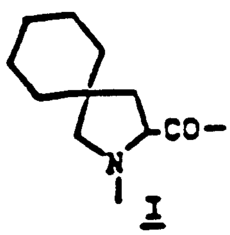
I

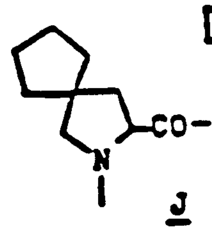
J

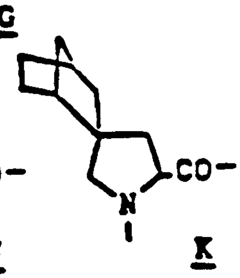
K

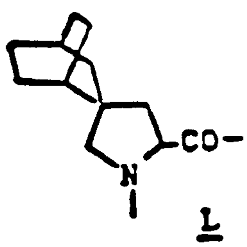
L

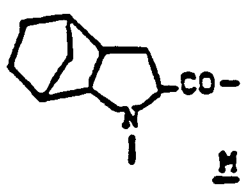
M

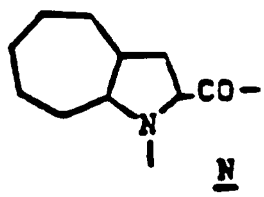
N

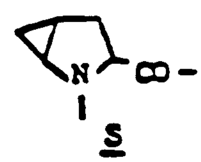
S

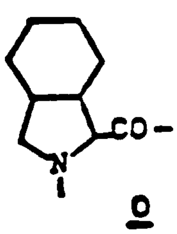
O

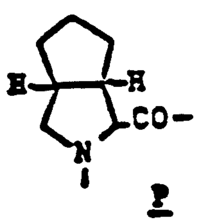
P

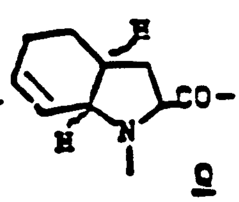
Q

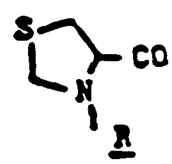
R

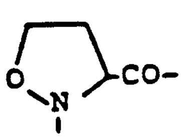
T

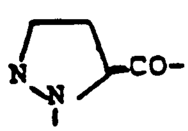
U

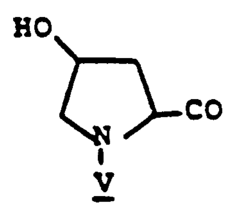
V

Die oben genannten Resten zugrundeliegenden Heterocyclen sind beispielsweise bekannt aus US-A 4 344 949, US-A 4 374 847, US-A 4 350 704, EP-A 50 800, EP-A 31 741, EP-A 51 020, EP-A 49 658, EP-A 49 605, EP-A 29 488, EP-A 46 953, EP-A 52 870, EP-A 271 865, DE-A 32 26 768, DE-A 31 51 690, DE-A 32 10 496, DE-A 32 11 397, DE-A 32 11 676, DE-A 32 27 055, DE-A 32 42 151, DE-A 32 46 503 und DE-A 32 46 757.

Ferner werden einige dieser Heterocyclen vorgeschlagen in DE-A 38 18 850.3.

Falls im Einzelfall nicht anders angegeben, kann Alkyl geradkettig oder verzweigt sein. Entsprechendes gilt für davon abgeleitete Reste wie Alkoxy, Aralkyl oder Alkanoyl.

($C_6$-$C_{12}$)-Aryl bedeutet vorzugsweise Phenyl, Naphtyl oder Biphenylyl. Entsprechend sind davon abgeleitete Reste, wie Aryloxy, Aralkyl oder Aroyl, zu formulieren.

Halo steht für Fluor, Chlor, Brom oder Jod, vorzugsweise für Chlor.

Als Salze kommen insbesondere Alkali- oder Erdalkalisalze, Salze mit physiologisch verträglichen Aminen und Salze mit anorganischen oder organischen Säuren wie z.B. HCl, HBr, $H_2SO_4$, $H_3PO_4$, Maleinsäure, Fumarsäure, Citronensäure, Weinsäure, Essigsäure in Frage.

Bevorzugt sind Peptide der Formel I, in welcher bedeuten:

B Arg, Orn oder Lys,
wobei die Guanidinogruppe bzw. die Aminogruppe der Seitenkette unsubstituiert ist oder durch ($C_1$-$C_8$)-Alkanoyl, ($C_7$-$C_{13}$)-Aryloyl, ($C_3$-$C_9$)-Heteroaryloyl, ($C_1$-$C_8$)-Alkylsulfonyl oder ($C_6$-$C_{12}$)-Arylsuffonyl substituiert sein kann, wobei die Aryl-, Heteroaryl-, Aryloyl-, Arylsulfonyl- und Heteroaryloylreste wie unter $a_2$) beschrieben mit gegebenenfalls 1, 2, 3 oder 4 gleichen oder verschiedenen Resten substituiert sein können;

E Phenylalanin, 2-Chlorphenylalanin, 3-Chlorphenylalanin, 4- Chlorphenylalanin, 2-Fluorphenylalanin, 3-Fluorphenylalanin, 4-Fluorphenylalanin, Tyrosin, O-Methyltyrosin oder $\beta$-(2-Thienyl)alanin;

K eine direkte Bindung;

M eine direkte Bindung.

Besonders bevorzugt sind Peptide der Formel I, in welcher bedeuten:

A Wasserstoff, (D)- oder (L)-H-Arg, (D)- oder (L)-H-Lys oder (D)- oder (L)-H-Orn;

B Arg, Orn oder Lys,
wobei die Guanidinogruppe bzw. die Aminogruppe der Seitenkette durch Wasserstoff, ($C_1$-$C_8$)-Alkanoyl, ($C_7$-$C_{13}$)-Aryloyl, ($C_3$-$C_9$)-Heteroaryloyl, ($C_1$-$C_8$)-Alkylsulfonyl oder ($C_6$-$C_{12}$)-Arylsuffonyl substituiert sein kann,
wobei die Aryl, Heteroaryl-, Aryloyl-, Arylsulfonyl- und Heteroaryloylreste gegebenenfalls mit 1, 2, 3 oder 4 gleichen oder verschiedenen Resten aus der Reihe Methyl, Methoxy und Halogen substituiert sein können;

C Pro-Pro-Gly, Hyp-Pro-Gly oder Pro-Hyp-Gly;

E Phe oder Thia;

F Ser, Hser, Lys, Leu, Val, Nle, Ile oder Thr;

K eine direkte Bindung;

M eine direkte Bindung;

G den Rest eines heterocyclischen Ringsystems der Formel IV, ausgewählt aus den Resten der Heterocyclen Pyrrolidin (A), Piperidin (B), Tetrahydroisochinolin (C), cis- oder trans-Decahydroisochinolin (D), cis-endo-Ootahydroindol (E), cis-exo-Octahydroindol (E), trans-Octahydroindol (E), cis-endo-, cis-exo-, trans-Octahydrocyclopentano[b]pyrrol (F), oder Hydroxyprolin (V);

F' Arg;

I OH.

Ganz besonders bevorzugt ist ein Peptid, ausgewählt aus der Gruppe bestehend aus:
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH,
H-(D)-Arg-Arg-Hyp-Pro-Gly-Phe-Ser-(D)-Tic-Oic-Arg-OH,
H-(D)-Arg-Arg-Pro-Pro-Gly-Phe-Ser-(D)-Tic-Oic-Arg-OH.

Die Erfindung betrifft weiterhin ein Verfahren zur Herstellung von Peptiden der Formel I, das dadurch gekennzeichnet ist, daß man

a) ein Fragment mit C-terminaler freier Carboxylgruppe oder dessen aktiviertes Derivat mit einem entsprechenden Fragment mit N-terminaler freier Aminogruppe umsetzt oder

b) das Peptid stufenweise aufbaut,

in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der

Formel I gegebenenfalls in ihr physiologisch verträgliches Salz überführt.

Die Peptide der vorliegenden Erfindung wurden nach allgemein bekannten Methoden der Peptidchemie, siehe z.B. Houben-Weyl, Methoden der organischen Chemie, Band 15/2, bevorzugt mittels Festphasensynthese wie z.B. von B. Merrifield, J.Am.Chem.Soc. 85, 2149 (1963) oder R. C. Sheppard, Int.J.Peptide Protein Res. 21, 118 (1983) beschrieben oder durch äquivalente bekannte Methoden hergestellt Als α-Aminoschutzgruppe werden Urethanschutzgruppen wie z.B. die tert.-Butyloxycarbonyl(Boc)- oder Fluorenylmethyloxycarbonyl(Fmoc)-Schutzgruppe verwendet. Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W.Greene, "Protective Groups in Organic Synthesis") zusätzlich geschützt, wobei in erster Linie
Arg(Tos), Arg(Mts), Arg(Mtr), Arg(PMC), Asp(OBzl), Asp(OBut), Cys(4-MeBzl), Cys(Acm), Cys(SBut), Glu-(OBzl), Glu(OBut), His(Tos), His(Fmoc), His(Dnp), His(Trt), Lys(Cl-Z), Lys(Boc), Met(O), Ser(Bzl), Ser(But), Thr(Bzl), Thr(But), Trp(Mts), Trp(CHO), Tyr(Br-Z), Tyr(Bzl) oder Tyr(But) eingesetzt werden.

Die Festphasensynthese beginnt am C-terminalen Ende des Peptids mit der Kupplung einer geschützten Aminosäure an ein entsprechendes Harz. Derartige Ausgangsmaterialien können durch Verknüpfung einer geschützten Aminosäure mit einem mit einer Chlormethyl-, Hydroxymethyl-, Benzhydrylamino(BHA)-, Methylbenzhydrylamino(MBHA)-Gruppe modifiziertem Polystyrol- oder Polyacrylamid-Harz über eine Ester- bzw. Amidbindung erhalten werden. Die als Trägermaterial verwendeten Harze sind kommerziell erhältlich. BHA- und MBHA-Harze werden für gewöhnlich verwendet, wenn das synthetisierte Peptid am C-Terminus eine freie Amidgruppe enthalten soll. Falls das Peptid eine sekundäre Amidgruppe am C-terminalen Ende enthalten soll wird ein Chlormethyl- bzw. Hydroxymethyl-Harz verwendet und die Abspaltung mit den entsprechenden Aminen durchgeführt. Will man z.B. das Ethylamid erhalten, kann das Peptid mit Ethylamin vom Harz abgespalten werden, wobei die Abspaltung der Seitenketten-Schutzgruppen nachfolgend durch andere geeignete Reagenzien, erfolgt. Sollen im Peptid die tert.-Butyl-Schutzgruppen der Aminosäure Seitenkette erhalten bleiben, so wird die Synthese mit der Fmoc-Schutzgruppe zur temporären Blockierung der α-Amino-Gruppe der Aminosäure unter Verwendung der z.B. bei R.C. Sheppard, J.Chem.Soc., Chem.Comm. 1982, 587 beschriebenen Methodik durchgeführt, wobei die Guanidino-Funktion des Arginins durch Protonierung mit Pyridinium-Perchlorat geschützt wird und der Schutz der anderen in der Seitenkette funktionalisierten Aminosäuren mit durch katalytische Transferhydrierung (A. Felix et al. J. Org. Chem. 13, 4194 (1978) oder durch Natrium in flüssigem Ammoniak (W.Roberts, J.Am.Chem.Soc. 76, 6203 (1954)) abspaltbaren Benzylschutzgruppen erfolgt.

Nach Abspaltung der Aminoschutzgruppe der an das Harz gekuppelten Aminosäure mit einem geeigneten Reagenz, wie z.B. Trifluoressigsäure in Methylenchlorid im Falle der Boc-Schutzgruppe oder einer 20 %igen Lösung von Piperidin in Dimethylformamid im Falle der Fmoc-Schutzgruppe, werden die nachfolgend geschützten Aminosäuren nacheinander in der gewünschten Reihenfolge aufgekuppelt.
Die intermediär entstehenden N-terminal geschützten Peptidharze werden vor der Verknüpfung mit dem nachfolgenden Aminosäurederivat durch die vorbeschriebenen Reagenzien entblockiert.

Als Kupplungsreagenz können alle möglichen in der Peptidsynthese verwendeten Aktivierungs-Reagenzien, siehe z.B. Houben-Weyl, Methoden der organischen Chemie, Band 15/2, verwendet werden, insbesondere aber Carbodiimide wie z.B. N,N'-Dicyclohexylcarbodiimid, N,N'-Diisopropylcarbodiimid oder N-Ethyl-N'-(3-dimethylaminopropyl)carbodiimid. Die Kupplung kann dabei direkt durch Addition von Aminosäurederivat mit dem Aktivierungsreagenz und gegebenenfalls einem die Racemisierung unterdrückenden Zusatz wie z.B. 1-Hydroxybenzotriazol (HOBt) (W. König, R. Geiger, Chem. Ber. 103, 708 (1970)) oder 3-Hydroxy-4-oxo-3,4-dihydrobenzotriazin (HOObt) (W. König, R.Geiger, Chem.Ber. 103, 2054 (1970) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Die Kupplung bzw. Aktivierung der Aminosäurederivate mit einem der oben genannten Aktivierungsreagenzien kann in Dimethylformamid, N-Methylpyrrolidon oder Methylenchlorid oder einer Mischung aus den genannten Lösungsmitteln durchgeführt werden. Das aktivierte Aminosäurederivat wird üblicherweise in einem 1,5 bis 4 fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockierung der α-Aminogruppe des Peptidharzes durchzuführen.

Der erfolgreiche Ablauf der Kupplungsreaktion kann mittels der Ninhydrin-Reaktion, wie z.B. von E.Kaiser et al. Anal. Biochem. 34 595 (1970) beschrieben, überprüft werden. Die Synthese kann auch automatisiert z.B. mit einem Peptid-Synthesizer Modell 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von

mit der Fmoc-Gruppe geschützten Aminosäurederivaten eingesetzt.

Nach Synthese der Peptide in der vorgehend beschriebenen Weise kann das Peptid vom Harz mit Reagenzien, wie z.B. flüssigem Fluorwasserstoff (bevorzugt bei den nach der Boc-Methode hergestellten Peptiden) oder Trifluoressigsäure (bevorzugt bei den nach der Fmoc-Methode synthetisierten Peptiden) abgespalten werden. Diese Reagenzien spalten nicht nur das Peptid vom Harz sondern auch die weiteren Seitenkettenschutzgruppen der Aminosäurederivate. Auf diese Weise erhält man außer bei der Verwendung von BHA- und MBHA-Harzen das Peptid in Form der freien Säure. Bei den BHA- bzw. MBHA-Harzen erhält man bei der Spaltung mit Fluorwasserstoff oder Trifluormethansulfonsäure das Peptid als Säureamid. Weitere Verfahren zur Herstellung von Peptidamiden sind in den deutschen Patentanmeldungen P 37 11 866.8 und P 37 43 620.1 beschrieben. Hier erfolgt die Abspaltung der Peptidamide vom Harz durch Behandlung mit in der Peptidsynthese üblicherweise verwendeten mittelstarken Säuren (z.B. Trifluoressigsäure) wobei als Kationenfänger Substanzen wie Phenol, Kresol, Thiokresol, Anisol, Thioanisol, Ethandithiol, Dimethylsulfid, Ethylmethylsulfid oder ähnliche in der Festphasensynthese übliche Kationenfänger einzeln oder eine Mischung von zwei oder mehr dieser Hilfsmittel zugesetzt werden. Die Trifluoressigsäure kann dabei auch durch geeignete Lösungsmittel, wie z.B. Methylenchlorid, verdünnt angewendet werden.

Falls die tert.-Butyl bzw. Benzylseitenketten-Schutzgruppen der Peptide erhalten bleiben sollen, wird die Abspaltung des an einem besonders modifizierten Trägerharz synthetisierten Peptides mit 1 % Trifluoressigsäure in Methylenchlorid durchgeführt, wie z.B. bei R.C. Sheppard J.Chem.Soc., Chem.Comm. 1982, 587 beschrieben. Sollen einzelne tert.-Butyl bzw. Benzylseitenketten-Schutzgruppen erhalten bleiben, so verwendet man eine geeignete Kombination der Synthese- und Abspaltmethoden.

Für die Synthese von Peptiden mit einer C-terminalen Amidgruppierung oder einer ω-Amino- bzw. ω-Guanidinoalkylgruppierung wird ebenfalls das von Sheppard beschriebene modifizierte Trägerharz verwendet. Nach der Synthese wird das in der Seitenkette vollgeschützte Peptid vom Harz abgespalten und anschließend in klassischer Lösungssynthese mit dem entsprechenden Amin bzw. ω-Aminoalkylamin oder ω-Guanidinoalkylamin umgesetzt, wobei gegebenenfalls vorhandene weitere funktionelle Gruppen in bekannter Weise temporär geschützt werden können.

Ein weiteres Verfahren zur Herstellung von Peptiden mit einer ω-Aminoalkylgruppierung ist in der deutschen Patentanmeldung P 36 35 670.0 beschrieben.

Die Peptide der vorliegende Erfindung wurden vorzugsweise unter Verwendung der Festphasentechnik nach zwei generellen Schutzgruppentaktiken synthetisiert:
Die Synthese erfolgte mit einem automatischen Peptidsynthesizer Modell 430 A der Fa. Applied Biosystems unter Verwendung von Boc- bzw. Fmoc-Schutzgruppen zur temporären Blockierung der α-Aminogruppe.

Bei Verwendung der Boc-Schutzgruppe wurden für die Synthese die vom Hersteller des Gerätes vorprogrammierten Synthesezyklen benutzt.

Die Synthese der Peptide mit einer freien Carboxylgruppe am C-terminalen Ende erfolgte an einem mit der entsprechenden Boc-Aminosäure funktionalisiertem 4-(Hydroxymethyl)-phenylacetsmidomethylpolystyrolharz (R.B. Merrifield, J.Org. Chem. 43, 2845 (1978)) der Fa. Applied Biosystems. Für die Herstellung der Peptidamide wurde ein MBHA-Harz derselben Firma verwendet.
Als Aktivierungsreagenzien dienten N,N'-Dicyclohexylcarbodiimid oder N,N'-Diisopropylcarbodiimid. Die Aktivierung erfolgte als symmetrisches Anhydrid, als HOBt-Ester oder HOObt-Ester in $CH_2Cl_2$, $CH_2Cl_2$ - DMF-Gemischen oder NMP. Für die Kupplung wurden 2-4 Äquivalente an aktiviertem Aminosäurederivat eingesetzt. Für Fälle in denen die Kupplung unvollständig verlief, wurde die Reaktion wiederholt.

Bei der Verwendung der Fmoc-Schutzgruppe zum temporären Schutz der α-Aminogruppe wurden für die Synthese mit dem automatischen Peptid-Synthesizer Modell 430A der Fa. Applied Biosystems eigene Syntheseprogramme eingegeben. Die Synthese erfolgte an einem p-Benzyloxybenzylalkohol-Harz (S. Wang, J.Am.Chem.Soc. 95, 1328 (1973)) der Fa. Bachem das nach bekannter Methode (E. Atherton et al. J.C.S.Chem. Comm. 1981, 336) mit der entsprechenden Aminosäure verestert war. Die Aktivierung der Aminosäurederivate als HOBt- oder HOObt-Ester erfolgte direkt in den vom Gerätehersteller gelieferten Aminosäurecartridges durch Zugabe einer Lösung von Diisopropylcarbodiimid in DMF zu der vorher eingewogenen Mischung aus Aminosäurederivat und HOBt oder HOObt. Ebenfalls eingesetzt werden können in Substanz hergestellte Fmoc-Aminosäure-OObt Ester wie sie in der europäischen Patentanmeldung 87107634.5 beschrieben sind. Die Abspaltung der Fmoc-Schutzgruppe erfolgte mit einer 20 %igen Lösung von Piperidin in DMF im Reaktionsgefäß. Der verwendete Überschuß an reaktivem Aminosäurederivat betrug 1,5 bis 2,5 Äquivalente. Falls die Kupplung nicht vollständig war, wurde sie wie bei der Boc-Methode wiederholt.

Die erfindungsgemäßen Peptide haben einzeln oder in Kombination eine bradykinin-antagonistische Wirkung, die in verschiedenen Modellen getestet werden kann (s. Handbook of Exp. Pharmacol. Vol. 25, Springer Verlag, 1970, S. 53 - 55), so z. B. am isolierten Rattenuterus, am Meerschweinchenileum oder an

der isolierten Pulmonalaterie des Meerschweinchens.

Für die Testung der erfindungsgemäßen Peptide an der isolierten Arteria pulmonalis werden Meerschweinchen (Dunkin Hartley) mit einem Gewicht von 400 - 450 g durch Genickschlag getötet.

Der Brustkorb wird geöffnet und die Arteria pulmonalis vorsichtig herauspräpariert. Das umliegende Gewebe wird sorgfältig entfernt und die Arteria pulmonalis in einem Winkel von 45° spiralig aufgeschnitten.

Der Gefäßstreifen von 2,5 cm Länge und 3 - 4 mm Breite wird in einem 10 ml fassenden Organbad, das mit Ringerlösung gefüllt ist, fixiert.

Zusammensetzung der Lösung in mmol/l

| | |
|---|---|
| NaCl | 154 |
| KCl | 5,6 |
| $CaCl_2$ | 1,9 |
| $NaHCO_3$ | 2,4 |
| Glukose | 5,0 |

Die Lösung wir mit 95 % $O_2$ und 5 % $CO_2$ durchperlt und auf 37° C erwärmt. Der pH beträgt 7,4, die Vorlast am Gefäßstreifen beträgt 1,0 g.

Die isotonische Kontraktionsänderungen werden mit einem Hebelvorsatz und einem HF-Modem (Wegmesser) von Hugo Sachs erfaßt und auf einen Kompensationsschreiber (BEC, Goerz Metrawatt SE 460) registriert.

Nach 1 Stunde Äquilibrierung wird mit dem Versuch begonnen. Nachdem die Gefäßstreifen ihre maximale Empfindlichkeit gegenüber 2 x $10^{-7}$ mol/l Bradykinin erreicht haben - Bradykinin führt zu einer Kontraktion der Gefäßstreifen - werden die Peptide in den Dosen 5 x $10^{-8}$ - 1 x $10^{-5}$ mol/l jeweils 10 Minuten einwirken lassen und nach erneuter Gabe von Bradykinin die Abnahme des Effektes von Bradykinin gegenüber der Kontrolle verglichen.

Zur Erkennung eines partialagonistischen Effektes werden die Peptide in den Dosen 1 x $10^{-5}$ - 1 x $10^{-3}$ mol/l verwendet.

Die aus den Dosiswirkungskurven errechneten $IC_{50}$-Werte der erfindungsgemäßen Peptide sind in der Tabelle 1 aufgeführt.

**Tabelle 1:**

| Verbindung | $IC_{50}$ [M] |
|---|---|
| H-(D)-Arg-Arg-Hyp-Pro-Gly-Phe-Ser-(D)-Tic-Phe-Arg-OH | $4{,}6 \cdot 10^{-6}$ |
| H-(D)-Arg-Arg-Hyp-Pro-Gly-Thia-Ser-(D)-Tic-Thia-Arg-OH | $2{,}1 \cdot 10^{-6}$ |
| H-(D)-Arg-Arg-Pro-Hyp-Gly-Phe-Ser-(D)-Tic-Phe-Arg-OH | $1{,}2 \cdot 10^{-5}$ |
| H-(D)-Arg-Arg-Hyp-Pro-Gly-Phe-Glo-(D)-Tic-Phe-Arg-OH | $2{,}4 \cdot 10^{-5}$ |
| H-(D)-Arg-Arg-Pro-Hyp-Gly-Phe-Ser-(D)-Tic-Phe-Arg(Mtr)-OH | $2{,}5 \cdot 10^{-5}$ |
| H-(D)-Arg-Arg-Hyp-Pro-Gly-Phe-Ser-(D)-Tic-Pro-Arg-OH | $2{,}5 \cdot 10^{-7}$ |
| H-(D)-Arg-Arg-Hyp-Pro-Gly-Thia-Ser-(D)-Tic-Pro-Arg-OH | $1{,}9 \cdot 10^{-7}$ |
| H-(D)-Arg-Arg-Hyp-Pro-Gly-Thia-Ser-(D)-Tic-Aoc-Arg-OH | $5{,}6 \cdot 10^{-8}$ |
| H-(D)-Arg-Arg-Hyp-Pro-Gly-Thia-Ser-β-Ala-(D)-Tic-Aoc-Arg-OH | $1{,}7 \cdot 10^{-6}$ |
| H-(D)-Arg-Arg-Hyp-Pro-Gly-Thia-Ser-Gly-(D)-Tic-Aoc-Arg-OH | $3{,}9 \cdot 10^{-7}$ |
| H-(D)-Arg-Arg-Hyp-Pro-Gly-Thia-Gly-(D)-Tic-(D,L)-Oic-Arg-OH | $3{,}2 \cdot 10^{-7}$ |
| H-(D)-Arg-(D)-Arg-Hyp-Pro-Gly-Thia-Ser-(D)-Tic-Aoc-Arg-OH | $4{,}8 \cdot 10^{-7}$ |
| H-(D)-Arg-Arg-Hyp-Pro-Gly-Thia-Ser-(D)-Tic-Tic-Arg-OH | $1{,}7 \cdot 10^{-7}$ |
| H-(D)-Arg-Arg-Pro-Hyp-Gly-Thia-Ser-(D)-Tic-Aoc-Arg-OH | $1{,}1 \cdot 10^{-8}$ |
| H-(D)-Arg-Arg-Pro-Hyp-Gly-Phe-Ser-(D)-Tic-Aoc-Arg-OH | $4{,}6 \cdot 10^{-8}$ |
| H-(D)-Tyr-Arg-Pro-Hyp-Gly-Thia-Ser-(D)-Tic-Aoc-Arg-OH | $6{,}2 \cdot 10^{-8}$ |
| H-(D)-Arg-Arg-Pro-Hyp-Gly-Thia-Ser-(D)-Tic-(D)-Dic-Arg-OH | $2{,}6 \cdot 10^{-5}$ |
| H-(D)-Arg-Arg-Pro-Hyp-Gly-Thia-Ser-(D)-Tic-Dic-Arg-OH | $5{,}4 \cdot 10^{-9}$ |
| H-(D)-Arg-Lys-Pro-Hyp-Gly-Phe-Ser-(D)-Tic-Aoc-Arg-OH | $3{,}2 \cdot 10^{-7}$ |
| H-(D)-Arg-Arg-Pro-Hyp-Gly-Phe-Ser-(D)-Tic-Oic-Arg-OH | $6{,}8 \cdot 10^{-9}$ |
| H-(D)-Arg-Arg-($NO_2$)-Pro-Hyp-Gly-Phe-Ser-(D)-Tic-Aoc-Arg-OH | $6{,}4 \cdot 10^{-8}$ |
| H-(D)-Arg-Arg-Pro-Pro-Gly-Thia-Ser-(D)-Tic-Dic-Arg-OH | $4{,}2 \cdot 10^{-9}$ |
| H-(D)-Arg-Pro-Hyp-Gly-Phe-Ser-(D)-Tic-Oic-Arg-OH | $3{,}4 \cdot 10^{-7}$ |

**Tabelle 1:**

| Verbindung | $IC_{50}$ [M] |
|---|---|
| H-Arg-(Tos)-Pro-Hyp-Gly-Phe-Ser-(D)-Tic-Dic-Arg-OH | $3{,}0 \cdot 10^{-8}$ |
| H-Arg-(Tos)-Pro-Hyp-Gly-Thia-Ser-(D)-Tic-Dic-Arg-OH | $1{,}8 \cdot 10^{-8}$ |

Der therapeutische Nutzen der erfindungsgemäßen Peptide umfaßt alle pathologischen Zustände, die durch Bradykinin und Bradykinin verwandte Peptide vermittelt, ausgelöst oder unterstützt werden. Dies beinhaltet u.a. Traumata, wie Wunden, Verbrennungen, Ausschläge, Erytheme, Ödeme, Angina, Arthritis, Asthma, Allergien, Rhinitis, Schock, Entzündungen, niedriger Blutdruck, Schmerz, Juckreiz und veränderte Sperma-Motilität.

Die Erfindung betrifft daher auch die Verwendung von Peptiden der Formel I als Heilmittel und pharmazeutische Präparate, die diese Verbindungen enthalten.

Pharmazeutische Präparate enthalten eine wirksame Menge des Wirkstoffs der Formel I - einzeln oder in Kombination - zusammen mit einem anorganischen oder organischen pharmazeutisch verwendbaren Trägerstoff.

Die Anwendung kann enteral, parenteral - wie z. B. subkutan, i. m. oder i. v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vozliegenden Erfindung werden in an sich bekannten Lösungs-, Misch-, Granulier- oder Dragierverfahren hergestellt.

Für die orale Anwendungsform oder zur Applikation auf die Schleimhäute werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Für die inhalative Anwendung können Vernebler oder Druckgaspackungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Einulsion gebracht.

Aufgrund der kurzen Halbwertszeiten einiger der beschriebenen Arzneistoffe in Körperflüssigkeiten ist der Einsatz von injizierbaren Retardzubereitungen sinnvoll. Als Arzneiformen können z. B. blige Kristallsuspensionen, Mikrokapseln, Rods oder Implantate verwendet werden, wobei die letzteren aus gewebeverträglichen Polymeren, insbesondere bioabbaubaren Polymeren, wie z. B. auf der Basis von Polymilchsäure-Polyglykolsäure-Copolymeren oder Humanalbumin aufgebaut sein können.

Ein geeigneter Dosisbereich für topische und inhalative Anwendungsformen sind Lösungen mit 0,01-5 mg/ml, bei systemischen Applikationsformen sind 0,01-10 mg/kg geeignet.

Verzeichnis der Abkürzungen:

Die für Aminosäuren verwendeten Abkürzungen entsprechen dem in der Peptidchemie üblichen drei Buchstaben-Code wie er in Europ. J. Biochem. 138, 9 (1984) beschrieben ist. Weitere verwendete Abkürzungen sind nachfolgend aufgelistet.

| | |
|---|---|
| Acm | Acetamidomethyl |
| ϵ-Ahx | ϵ-Aminohexanoyl |
| Aoc | cis, endo-2-Azabicyclo[3.3.0]octan-3-S-carbonyl |
| Boc | tert.-Butyloxycarbonyl |
| But | tert.-Butyl |
| Bzl | Benzyl |
| Cl-Z | 4-Chlor-benzyloxycarbonyl |
| DMF | Dimethylformamid |
| Dnp | 2,4-Dinitrophenyl |
| Fmoc | 9-Fluorenylmethyloxycarbonyl |
| Me | Methyl |
| 4-Mebzl | 4-Methylbenzyl |

| | |
|---|---|
| Mtr | 4-Methoxy-2,3,6-trimethylphenyllsulfonyl |
| Mts | Mesitylen-2-sulfonyl |
| NMP | N-Methylpyrrolidin |
| Oic | cis-endo-Octahydroindol-2-carbonyl |
| Opr | Isoxazolidin-3-ylcarbonyl |
| Pmc | 2,2,5,7,8-Pentamethylchroman-6-sulfonyl |
| TFA | Trifluoressigsäure |
| Tcs | 4-Methylphenylsulfonyl |
| Thia | 2-Thienylalanyl |
| Tic | 1,2,3,4-Tetrahydroisochinolin-3-ylcarbonyl |
| Trt | Trityl |

Die folgenden Beispiele sollen die bevorzugten Methoden zur Festphasen-Synthese der erfindungsgemäßen Peptide verdeutlichen, ohne daß die Erfindung darauf eingeschränkt wäre.

Man verwendete folgende Aminosäure-Derivate:
Fmoc-Arg(Mtr)-OH, Boc-(D)-Arg-OH, Fmoc-Arg(Pmc)-OH, Fmoc-Hyp-OH, Fmoc-Pro-OObt, Fmoc-Gly-OObt, Fmoc-Phe-OObt, Fmoc-Ser(tBu)-OObt, Fmoc-(D)-Tic-OH, Fmoc-Gln-OH, Fmoc-Aoc-OH, Fmoc-Thia-OH, Fmoc-Opr-OH, Fmoc-(D)-Asn-OH, Fmoc-$\beta$-Ala-OH, Fmoc-Oic-OH.

**Beispiel 1:**

H-(D)-Arg-Arg-Hyp-Pro-Gly-Phe-Ser-(D)-Tic-Phe-Arg-OH
wurde mit einem Peptid-Synthesizer Modell 430 A der Fa.

Applied Biosystems unter Verwendung der Fmoc-Methode an einem mit Fmoc-Arg(Mtr)-OH veresterten p-Benzyloxybenzylalkohol-Harz der Fa. Novabiochem (Beladung ca. 0,5 mmol/g Harz) stufenweise aufgebaut. Es wurde 1 g des Harzes eingesetzt und die Synthese mit Hilfe eines für die Fmoc-Methode modifizierten Synthese-Programmes durchgeführt.

In die Cartridges des Synthesizers wurden jeweils 1 mmol der Aminosäurederivate mit freier Carboxylgruppe zusammen mit 0,95 mmol HOObt eingewogen. Die Voraktivierung dieser Aminosäuren erfolgte direkt in den Cartridges durch Lösen in 4 ml DMF und Zugabe von 2 ml einer 0,55 mol Lösung von Diisopropylcarbodiimid in DMF.
Die HOObt-Ester der anderen Aminosäuren wurden in 6 ml NMP gelöst und dann ebenso wie die in situ voraktivierten Aminosäuren an das vorher mit 20 % Piperidin in DMF entblockierte Harz gekuppelt. Nach beendeter Synthese wurde das Peptid unter gleichzeitiger Entfernung der Seitenkettenschutzgruppen mit Trifluoressigsäure unter Verwendung von Thioanisol und Ethandithiol als Kationenfänger vom Harz abgespalten. Der nach Abziehen der Trifluoressigsäure erhaltene Rückstand wurde mehrfach mit Essigester digeriert und zentrifugiert. Der verbliebene Rückstand wurde an ®Sephadex LH 20 mit 10 %iger Essigsäure chromatographiert. Die das reine Peptid enthaltenden Fraktionen wurden vereinigt und gefriergetrocknet
MS(FAB) : 1294 (M + H)
Die Peptide der nachfolgenden Beispiele 2 bis 24 wurde analog Beispiel 1 dargestellt und gereinigt.

**Beispiel 2:**

H-(D)-Arg-Arg-Hyp-Pro-Gly-Phe-(D)-Ser-(D)-Tic-Phe-Arg-OH
MS(FAB) : 1294 (M + H)

**Beispiel 3:**

H-(D)-Arg-Arg-Hyp-Pro-Gly-Thia-Ser-(D)-Tic-Thia-Arg-OH
MS(FAB) : 1306 (M + H)

**Beispiel 4:**

H-(D)-Arg-Arg-Pro-Hyp-Gly-Phe-Ser-(D)-Tic-Phe-Arg-OH
MS(FAB) : 1294 (M + H)

**Beispiel 5:**

H-(D)-Arg-Arg-Hyp-Pro-Gly-Phe-Gln-(D)-Tic-Phe-Arg-OH
MS(FAB) : 1335 (M + H)

**Beispiel 6:**

H-(D)-Arg-Arg-Hyp-Pro-Gly-Phe-Ser-(D)-Tic-Pro-Arg-OH
MS(FAB) : 1244 (M + H)

**Beispiel 7:**

H-(D)-Arg-Arg-Hyp-Pro-Gly-Phe-Trp-(D)-Tic-Phe-Arg-OH
MS(FAB) : 1393 (M + H)

**Beispiel 8:**

H-(D)-Arg-Arg-Hyp-Pro-Gly-Thia-Ser-(D)-Tic-Pro-Arg-OH
MS(FAB) : 1250 (M + H)

**Beispiel 9:**

H-(D)-Arg-Arg-Hyp-Pro-Gly-Thia-(D)-Asn-(D)-Tic-Thia-Arg-OH
MS(FAB) : 1333 (M + H)

**Beispiel 10:**

H-(D)-Arg-Arg-Hyp-Pro-Gly-Thia-Opr-(D)-Tic-Thia-Arg-OH
MS(FAB) : 1301 (M + H)

**Beispiel 11:**

H-(D)-Arg-Arg-Hyp-Pro-Gly-Thia-(D)-Gln-(D)-Tic-Thia-Arg-OH
MS(FAB) : 1347 (M + H)

**Beispiel 12:**

H-(D)-Arg-Arg-Hyp-Pro-Gly-Thia-Ser-Gly-(D)-Tic-Pro-Arg-OH
MS(FAB) : 1307 (M + H)

**Beispiel 13:**

H-(D)-Arg-Arg-Hyp-Pro-Gly-Thia-Ser-(D)-Tic-Pro-Phe-OH
MS(FAB) : 1241 (M + H)

**Beispiel 14:**

H-(D)-Arg-Arg-Hyp-Pro-Gly-Thia-Ser-(D)-Tic-Pro-Phe-Arg-OH
MS(FAB) : 1397 (M + H)

**Beispiel 15:**

H-(D)-Arg-Arg-Hyp-Pro-Gly-Thia-Ser-β-Ala-(D)-Tic-Pro-Arg-OH
MS(FAB) : 1321 (M + H)

**Beispiel 16:**

H-(D)-Arg-Arg-Hyp-Pro-Gly-Thia-Gly-(D)-Tic-Pro-Arg-OH
MS(FAB) : 1220 (M + H)

**Beispiel 17:**

H-(D)-Arg-Arg-Aoc-Pro-Gly-Thia-Ser-(D)-Tic-Thia-Arg-OH
MS(FAB) : 1330 (M + H)

**Beispiel 18:**

H-(D)-Arg-Arg-Pro-Aoc-Gly-Thia-Ser-(D)-Tic-Thia-Arg-OH
MS(FAB) : 1330 (M + H)

**Beispiel 19:**

H-(D)-Arg-Arg-Hyp-Pro-Gly-Thia-Ser-(D)-Tic-Aoc-Arg-OH
MS(FAB) : 1290 (M + H)

**Beispiel 20:**

H-(D)-Arg-Arg-Opr-Pro-Gly-Thia-Ser-(D)-Tic-Pro-Arg-OH
MS(FAB) : 1236 (M + H)

**Beispiel 21:**

H-(D)-Arg-Arg-Pro-Opr-Gly-Thia-Ser-(D)-Tic-Pro-Arg-OH
MS(FAB) : 1236 (M + H)

**Beispiel 22:**

H-(D)-Arg-Arg-Hyp-Pro-Gly-Thia-Ser-(D)-Tic-Opr-Arg-OH
MS(FAB) : 1252 (M + H)

**Beispiel 23:**

H-(D)-Arg-(D)-Arg-Hyp-Pro-Gly-Thia-Ser-(D)-Tic-Aoc-Arg-OH
MS(FAB) : 1290 (M + H)

**Beispiel 24:**

H-(D)-Arg-Arg-Pro-Hyp-Gly-Thia-Ser-(D)-Tic-Aoc-Arg-OH
MS(FAB) : 1290 (M + H)

**Beispiele 25 - 27:**

H-(D)-Arg-Arg(Mtr)-Pro-Hyp-Gly-Phe-Ser-(D)-Tic-Phe-Arg-OHund
H-(D)-Arg-Arg-Pro-Hyp-Gly-Phe-Ser-(D)-Tic-Phe-Arg(Mtr)-OHund
H-(D)-Arg-Arg(Mtr)-Pro-Hyp-Gly-Phe-Ser-(D)-Tic-Phe-Arg(Mtr)-OH
werden analog Beispiel 1 dargestellt, wobei die Abspaltung der Seitenkettenschutzgruppen und des Peptides vom Harz mittels Trifluoressigsäure auf 30 Minuten bei Raumtemperatur beschränkt wurde. Unter den so gewählten Bedingungen findet nur eine vernachlässigbare Abspaltung der Mtr-Schutzgruppe am Arginin statt. Die partiell deblockierten Peptide werden durch Chromatographie an Reverse-Phase-Material aufgetrennt und gereinigt.

25: H-(D)-Arg-Arg(Mtr)-Pro-Hyp-Gly-Phe-Ser-(D)-Tic-Phe-Arg-OH
MS(FAB): 1506 (M + H)

26: H-(D)-Arg-Arg(Mtr)-Pro-Hyp-Gly-Phe-Ser-(D)-Tic-Phe-Arg(Mtr)-OH
MS(FAB): 1718 (M + H)
27: H-(D)-Arg-Arg-Pro-Hyp-Gly-Phe-Ser-(D)-Tic-Phe-Arg(Mtr)-OH
MS(FAB): 1506 (M + H)

Die Peptide der nachfolgenden Beispiple 28 - 31 wurden analog der Beispiele 25 - 27 dargestellt und gereinigt.

**Beispiel 28:**

H-(D)-Arg-Arg(Mtr)-Hyp-Pro-Gly-Thia-Ser-(D)-Tic-Pro-Arg-OH
MS(FAB) : 1462 (M + H)

**Beispiel 29:**

H-(D)-Arg-Arg-Hyp-Pro-Gly-Thia-Ser-(D)-Tic-Pro-Arg(Mtr)-OH
MS(FAB) : 1462 (M + H)

**Beispiel 30:**

H-(D)-Arg-Arg(Mtr)-Hyp-Pro-Gly-Thia-Ser-(D)-Tic-Pro-Phe-OH
MS(FAB) : 1453 (M + H)

**Beispiel 31:**

H-(D)-Arg-Arg(Mtr)-Hyp-Pro-Gly-Thia-Ser-(D)-Tic-Aoc-Arg-OH
MS(FAB) : 1502 (M + H)

**Beispiel 32:**

H-Arg-Hyp-Pro-Gly-Phe-Ser-(D)-Tic-Phe-NH-$(CH_2)_4$-$NH_2$.

Die Peptidsynthese erfolgte an 1 g eines Aminomethylharzes, das mit einer in der EP-A 264 802 beschriebenen Ankergruppe vom Typ

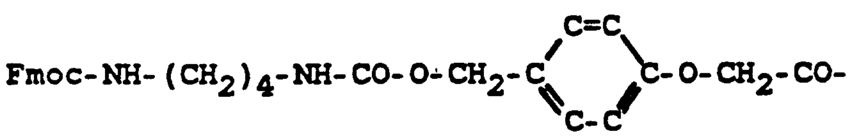

modifiziert war, unter Verwendung von Fmoc-Aminosäure-OObt Estern mit einem automatischen Peptidsynthesizer (Modell 430A der Fa. Applied Biosystems) und selbst modifizierten Syntheseprogrsmmen. Dazu wurden jeweils 1 mMol des entsprechenden Aminosäurederivates in die vom Hersteller gelieferten Cartridges eingewogen, Fmoc-Arg(Mtr)-OH, Fmoc-Hyp-OH und Fmoc-(D)-Tic-OH wurden zusammen mit 0,95 mMol HOObt in die Cartridges eingewogen. Die in situVoraktivierung dieser Aminosäuren erfolgte direkt in den Cartridges durch Lösen in 4 ml DMF und Zugabe von 2 ml einer 0,55 M Lösung von Diisopropylcarbodiimid in DMF. Die HOObt-Ester der anderen Aminosäuren wurden in 6 ml NMP gelöst und dann ebenso wie die in situ voraktivierten Aminosäuren an das vorher mit 20 % Piperidin in DMF entblockierte Harz gekuppelt, wobei die in situ aktivierten Aminosäuren doppelt gekuppelt wurden. Nach beendeter Synthese wurde das Peptid-4-amino-butylamid unter gleichzeitiger Entfernung der Seitenkettenschutzgruppen mit Trifluoressigsäure, die Thioanisol und m-Kresol als Kationenfänger enthielt, vom Harz abgespalten. Der nach Abziehen der Trifluoressigsäure erhaltene Rückstand wurde mehrfach mit Essigester digeriert und zentrifugiert. Das verbliebene Rohpeptid wurde an ®Sephadex G25 mit 1N Essigsäure chromatographiert. Die das reine Peptid enthaltenden Fraktionen wurden vereinigt und gefriergetrocknet.

Analog Beispiel 32 wurden die Verbindungen der Beispiele 33 - 35 hergestellt:

**Beispiel 33:**

H-D-Arg-Arg-Hyp-Pro-Gly-Phe-Ser-(D)-Tic-Phe-NH-$(CH_2)_4$-$NH_2$

**Beispiel 34:**

HOOC-$(CH_2)_2$-CO-Arg-Hyp-Pro-Gly-Phe-Ser-(D)-Tic-Phe-NH-$(CH_2)_4$-$NH_2$

**Beispiel 35:**

HOOC-$(CH_2)_2$-CO-(D)-Arg-Hyp-Pro-Gly-Phe-Ser-(D)-Tic-Phe-NH-$(CH_2)_4$-$NH_2$
Die Beispiele 36 bis wurden nach der unter Beispiel 1 beschriebenen Methode synthetisiert.

**Beispiel 36:**

H-(D)-Arg-Arg-Hyp-Pro-Gly-Thia-Ser-Gly-(D)-Tic-Pro-Arg-OH
MS(FAB) : 1307 (M + H)

**Beispiel 37:**

H-(D)-Arg-Arg-Pro-Hyp-Gly-Thin-Ser-Gly-(D)-Tic-Pro-Arg-OH
MS(FAB) : 1307 (M + H)

**Beispiel 38:**

H-(D)-Arg-Arg-Hyp-Pro-Gly-Thia-Ser-(D)-Tic-Pro-Phe-OH
MS(FAB) : 1241 (M + H)

**Beispiel 39:**

H-(D)-Arg-Arg-Hyp-Pro-Gly-Thia-Ser-$\beta$-Ala-(D)-Tic-Aoc-Arg-OH
MS(FAB) : 1361 (M + H)

**Beispiel 40:**

H-(D)-Arg-Arg-Pro-Hyp-Gly-Thia-Ser-$\beta$-Ala-(D)-Tic-Aoc-Arg-OH
MS(FAB) : 1361 (M + H)

**Beispiel 41:**

H-(D)-Arg-Arg-Hyp-Pro-Gly-Thia-Ser-(D)-Tic-Pro-Phe-Arg-OH
MS(FAB) : 1397 (M + H)

**Beispiel 42:**

H-(D)-Arg-Arg-Pro-Hyp-Gly-Thia-Ser-(D)-Tic-Pro-Phe-Arg-OH
MS(FAB) : 1397 (M + H)

**Beispiel 43:**

H-(D)-Arg-Arg-Pro-Hyp-Gly-Thia-Gly-(D)-Tic-Aoc-Arg-OH
MS(FAB) : 1260 (M + H)

**Beispiel 44:**

H-(D)-Arg-Arg-Hyp-Pro-Gly-Thia-Gly-(D)-Tic-Aoc-Arg-OH
MS(FAB) : 1260 (M + H)

**Beispiel 45:**

H-(D)-Arg-(D)-Arg-Hyp-Pro-Gly-Thia-Ser-(D)-Tic-Aoc-Arg-OH
MS(FAB) : 1290 (M + H)

**Beispiel 46:**

H-(D)-Arg-(D)-Arg-Pro-Hyp-Gly-Thia-Ser-(D)-Tic-Aoc-Arg-OH
MS(FAB) : 1290 (M + H)

**Beispiel 47:**

H-(D)-Arg-Arg-Hyp-Pro-Gly-Thia-Ser-(D)-Tic-Tic-Arg-OH
MS(FAB) : 1312 (M + H)

**Beispiel 48:**

H-(D)-Arg-Arg-Pro-Hyp-Gly-Thia-Ser-(D)-Tic-Tic-Arg-OH
MS(FAB) : 1312 (M + H)

**Beispiel 49:**

H-(D)-Arg-Arg-Pro-Pro-Gly-Thia-Ser-(D)-Tic-Aoc-Arg-OH
MS(FAB) : 1274 (M + H)

**Beispiel 50:**

H-(D)-Arg-Arg-Hyp-Pro-Gly-Thia-(D)-Tic-Aoc-Arg-OH
MS(FAB) : 1203 (M + H)

**Beispiel 51:**

H-(D)-Arg-Arg-Hyp-Pro-Gly-Aoc-Ser-(D)-Tic-Aoc-Arg-OH
MS(FAB) : 1274 (M + H)

**Beispiel 52:**

H-(D)-Arg-Arg-Hyp-Pro-Gly-Thia-β-Ala-(D)-Tic-Aoc-Arg-OH
MS(FAB) : 1274 (M + H)

**Beispiel 53:**

H-(D)-Arg-Arg-Pro-Hyp-Gly-Thia-β-Ala-(D)-Tic-Aoc-Arg-OH
MS(FAB) : 1274 (M + H)

**Beispiel 54:**

H-(D)-Arg-Arg-Hyp-Pro-Gly-Asp-Ser-(D)-Tic-Aoc-Arg-OH
MS(FAB) : 1252 (M + H)

**Beispiel 55:**

H-(D)-Arg-Arg-Pro-Hyp-Gly-Asp-Ser-(D)-Tic-Aoc-Arg-OH
MS(FAB) : 1252 (M + H)

**Beispiel 56:**

H-(D)-Arg-Arg-Hyp-Pro-Gly-Trp-Ser-(D)-Tic-Aoc-Arg-OH
MS(FAB) : 1323,7 (M + H)

**Beispiel 57:**

H-(D)-Tyr-Arg-Pro-Hyp-Gly-Thia-Ser-(D)-Tic-Aoc-Arg-OH
MS(FAB) : 1297,7 (M + H)

**Beispiel 58:**

H-(D)-Arg-Arg-Pro-Hyp-Gly-Thia-Ser-(D)-Tic-(D)-Oic-Arg-OH
MS(FAB) : 1304,6 (M + H)

**Beispiel 59:**

H-(D)-Arg-Arg-Pro-Hyp-Gly-Thia-Ser-(D)-Tic-Oic-Arg-OH
MS(FAB) : 1304,6 (M + H)

**Beispiel 60:**

H-(D)-Arg-Arg-Pro-Pro-Gly-Thia-Ser-(D)-Tic-Oic-Arg-OH
MS(FAB) : 1289 (M + H)

**Beispiel 61:**

H-(D)-Arg-Lys-Pro-Hyp-Gly-Thia-Ser-(D)-Tic-Aoc-Arg-OH
MS(FAB) : 1262 (M + H)

**Beispiel 62:**

H-(D)-Arg-Lys-Pro-Hyp-Gly-Thia-Ser-(D)-Tic-Oic-Arg-OH
MS(FAB) : 1276 (M + H)

**Beispiel 63:**

H-(D)-Arg-Lys-Pro-Pro-Gly-Thia-Ser-(D)-Tic-Oic-Arg-OH
MS(FAB) : 1260 (M + H)

**Beispiel 64:**

H-(D)-Arg-Arg-Pro-Hyp-Gly-Phe-Ser-(D)-Tic-Oic-Arg-OH
MS(FAB) : 1298 (M + H)

**Beispiel 65:**

H-(D)-Arg-Arg-Hyp-Pro-Gly-Phe-Ser-(D)-Tic-Oic-Arg-OH
MS(FAB) : 1298 (M + H)

**Beispiel 66:**

H-(D)-Arg-Arg-Pro-Pro-Gly-Phe-Ser-(D)-Tic-Oic-Arg-OH
MS(FAB) : 1282 (M + H)

**Beispiel 67:**

H-(D)-Arg-Arg($NO_2$)-Pro-Hyp-Gly-Phe-Ser-(D)-Tic-Aoc-Arg-OH
MS(FAB) : 1329,7 (M + H)

**Beispiel 68:**

H-(D)-Arg-Arg($NO_2$)-Pro-Hyp-Gly-Phe-Ser-(D)-Tic-Oic-Arg-OH
MS(FAB): 1343 (M + H)

**Beispiel 69:**

H-(D)-Arg-Arg($NO_2$)-Pro-Pro-Gly-Phe-Ser-(D)-Tic-Oic-Arg-OH
MS(FAB) : 1327 (M + H)

**Beispiel 70:**

H-(D)-Arg-Arg($NO_2$)-Pro-Pro-Gly-Thia-Ser-(D)-Tic-Oic-Arg-OH
MS(FAB) : 1333 (M + H)

**Beispiel 71:**

H-(D)-Arg-Arg($NO_2$)-Pro-Hyp-Gly-Thia-Ser-(D)-Tic-Oic-Arg-OH
MS(FAB) : 1349 (M + H)

**Beispiel 72:**

H-Arg(Tos)-Pro-Hyp-Gly-Thia-Ser-D-Tic-Oic-Arg-OH
MS(FAB) : 1302 (M + H)

**Beispiel 73:**

H-Arg-Pro-Hyp-Gly-Phe-Ser-D-Tic-Oic-Arg-OH
MS(FAB) : 1142 (M + H)

**Beispiel 74:**

H-Lys(-CO-NH-$C_6H_5$)-Pro-Hyp-Gly-Phe-Ser-D-Tic-Oic-Arg-OH
MS(FAB) : 1233 (M + H)

**Beispiel 75:**

H-Arg(Tos)-Pro-Hyp-Gly-Phe-Ser-D-Tic-Oic-Arg-OH
MS(FAB) : 1296 (M + H)

**Beispiel 76:**

H-Lys(Nicotinoyl)-Pro-Hyp-Gly-Phe-Ser-D-Tic-Oic-Arg-OH
MS(FAB) : 1219 (M + H)

**Beispiel 77:**

H-Arg(Tos)-Pro-Hyp-Gly-Phe-Ser-D-Tic-Aoc-Arg-OH
MS(FAB) : 1282 (M + H)

**Beispiel 78:**

Ac-Arg(Tos)-Pro-Hyp-Gly-Phe-Ser-D-Tic-Aoc-Arg-OH
MS(FAB) : 1324 (M + H)

**Beispiel 79:**

H-D-Arg-Arg(Tos)-Pro-Hyp-Gly-Phe-Ser-D-Tic-Aoc-Arg-OH
MS(FAB) : 1438 (M + H)

**Beispiel 80:**

H-Arg(Tos)-Hyp-Pro-Gly-Thia-Ser-D-Tic-Oic-Arg-OH
MS(FAB) : 1302 (M + H)

**Beispiel 81:**

H-Arg-Hyp-Pro-Gly-Phe-Ser-D-Tic-Oic-Arg-OH
MS(FAB) : 1142 (M + H)

**Beispiel 82:**

H-Lys(-CO-NH-$C_6H_5$)-Hyp-Pro-Gly-Phe-Ser-D-Tic-Oic-Arg-OH
MS(FAB) : 1233 (M + H)

**Beispiel 83:**

H-Arg(Tos)-Hyp-Pro-Gly-Phe-Ser-D-Tic-Oic-Arg-OH
MS(FAB) : 1296 (M + H)

**Beispiel 84:**

H-Lys(Nicotinoyl)-Hyp-Pro-Gly-Phe-Ser-D-Tic-Oic-Arg-OH
MS(FAB) : 1219 (M + H)

**Beispiel 85:**

H-Arg(Tos)-Hyp-Pro-Gly-Phe-Ser-D-Tic-Aoc-Arg-OH
MS(FAB) : 1282 (M + H)

**Beispiel 86:**

Ac-Arg(Tos)-Hyp-Pro-Gly-Phe-Ser-D-Tic-Aoc-Arg-OH
MS(FAB) : 1324 (M + H)

**Beispiel 87:**

H-D-Arg-Arg(Tos)-Hyp-Pro-Gly-Phe-Ser-D-Tic-Aoc-Arg-OH
MS(FAB) : 1438 (M + H)

**Beispiel 88:**

H-Arg(Tos)-Pro-Pro-Gly-Thia-Ser-D-Tic-Oic-Arg-OH
MS(FAB) : 1286 (M + H)

**Beispiel 89:**

H-Arg-Pro-Pro-Gly-Phe-Ser-D-Tic-Oic-Arg-OH
MS(FAB) : 1126 (M + H)

**Beispiel 90:**

H-Lys(-CO-NH-$C_6H_5$)-Pro-Pro-Gly-Phe-Ser-D-Tic-Oic-Arg-OH
MS(FAB) : 1217 (M + H)

**Beispiel 91:**

H-Arg(Tos)-Pro-Pro-Gly-Phe-Ser-D-Tic-Oic-Arg-OH
MS(FAB) : 1280 (M + H)

**Beispiel 92:**

H-Lys(Nicotinoyl)-Pro-Pro-Gly-Phe-Ser-D-Tic-Oic-Arg-OH
MS(FAB) : 1203 (M + H)

**Beispiel 93:**

H-Arg(Tos)-Pro-Pro-Gly-Phe-Ser-D-Tic-Aoc-Arg-OH
MS(FAB) : 1266 (M + H)

**Beispiel 94:**

Ac-Arg(Tos)-Pro-Pro-Gly-Phe-Ser-D-Tic-Aoc-Arg-OH
MS(FAB) : 1308 (M + H)

**Beispiel 95:**

H-D-Arg-Arg(Tos)-Pro-Pro-Gly-Phe-Ser-D-Tic-Aoc-Arg-OH
MS(FAB) : 1422 (M + H)

**Beispiel 96:**

H-Arg-Pro-Hyp-Gly-Thia-Ser-D-Tic-Oic-Arg-OH
MS(FAB) : 1148 (M + H)

**Beispiel 97:**

H-Lys(-CO-NH-$C_6H_5$)-Pro-Hyp-Gly-Thia-Ser-D-Tic-Oic-Arg-OH
MS(FAB) : 1239 (M + H)

**Beispiel 98:**

H-Lys(Nicotinoyl)-Pro-Hyp-Gly-Thia-Ser-D-Tic-Oic-Arg-OH
MS(FAB) : 1225 (M + H)

**Beispiel 99:**

H-Arg(Tos)-Pro-Hyp-Gly-Thia-Ser-D-Tic-Aoc-Arg-OH
MS(FAB) : 1288 (M + H)

**Beispiel 100:**

Ac-Arg(Tos)-Pro-Hyp-Gly-Thia-Ser-D-Tic-Aoc-Arg-OH
MS(FAB) : 1330 (M + H)

**Beispiel 101:**

H-D-Arg-Arg(Tos)-Pro-Hyp-Gly-Thia-Ser-D-Tic-Aoc-Arg-OH
MS(FAB) : 1444 (M + H)

**Beispiel 102:**

H-Arg-Hyp-Pro-Gly-Thia-Ser-D-Tic-Oic-Arg-OH
MS(FAB) : 1148 (M + H)

**Beispiel 103:**

H-Lys(-CO-NH-$C_6H_5$)-Hyp-Pro-Gly-Thia-Ser-D-Tic-Oic-Arg-OH
MS(FAB) : 1239 (M + H)

**Beispiel 104:**

H-Lys(Nicotinoyl)-Hyp-Pro-Gly-Thia-Ser-D-Tic-Oic-Arg-OH
MS(FAB) : 1225 (M + H)

**Beispiel 105:**

H-Arg(Tos)-Hyp-Pro-Gly-Thia-Ser-D-Tic-Aoc-Arg-OH
MS(FAB) : 1288 (M + H)

**Beispiel 106:**

Ac-Arg(Tos)-Hyp-Pro-Gly-Thia-Ser-D-Tic-Aoc-Arg-OH
MS(FAB) : 1330 (M + H)

**Beispiel 107:**

H-D-Arg-Arg(Tos)-Hyp-Pro-Gly-Thia-Ser-D-Tic-Aoc-Arg-OH
MS(FAB) : 1440 (M + H)

**Beispiel 108:**

H-Lys(-CO-NH-$C_6H_5$)-Pro-Pro-Gly-Thia-Ser-D-Tic-Oic-Arg-OH
MS(FAB) : 1225 (M + H)

**Beispiel 109:**

H-Lys(Nicotinoyl)-Pro-Pro-Gly-Thia-Ser-D-Tic-Oic-Arg-OH
MS(FAB) : 1209 (M + H)

**Beispiel 110:**

H-Arg(Tos)-Pro-Pro-Gly-Thia-Ser-D-Tic-Aoc-Arg-OH
MS(FAB): 1272 (M + H)

**Beispiel 111:**

Ac-Arg(Tos)-Pro-Pro-Gly-Thia-Ser-D-Tic-Aoc-Arg-OH
MS(FAB) : 1314 (M + H)

**Beispiel 112:**

H-D-Arg-Arg(Tos)-Pro-Pro-Gly-Thia-Ser-D-Tic-Aoc-Arg-OH
MS(FAB) : 1428 (M + H)

**Beispiel 113:**

H-D-Arg-Lys(Nicotinoyl)-Pro-Pro-Gly-Thia-Ser-D-Tic-Oic-Arg-OH
MS(FAB) : 1365 (M + H)

**Beispiel 114:**

H-D-Arg-Lys(-CO-NH-$C_6H_5$)-Pro-Pro-Gly-Thia-Ser-D-Tic-Oic-Arg-OH
MS(FAB) : 1379 (M + H)

**Beispiel 115:**

H-D-Arg-Arg(Tos)-Pro-Pro-Gly-Thia-Ser-D-Tic-Oic-Arg-OH
MS(FAB) : 1442 (M + H)

**Beispiel 116:**

H-Lys-Lys-(Nicotinoyl)-Pro-Pro-Gly-Thia-Ser-D-Tic-Oic-Arg-OH
MS(FAB) : 1337 (M + H)

**Beispiel 117:**

H-Lys-Lys(-CO-NH-$C_6H_5$)-Pro-Pro-Gly-Thia-Ser-D-Tic-Oic-Arg-OH
MS(FAB) : 1351 (M + H)

**Beispiel 118:**

H-Lys-Arg(Tos)-Pro-Pro-Gly-Thia-Ser-D-Tic-Oic-Arg-OH
MS(FAB) : 1414 (M + H)

**Beispiel 119:**

H-D-Arg-Lys(Nicotinoyl)-Pro-Hyp-Gly-Thia-Ser-D-Tic-Oic-Arg-OH
MS(FAB) : 1381 (M + H)

**Beispiel 120:**

H-D-Arg-Lys-(CO-NH-$C_6H_5$)-Pro-Hyp-Gly-Thia-Ser-D-Tic-Oic-Arg-OH
MS(FAB) : 1395 (M + H)

**Beispiel 121:**

H-D-Arg-Arg(Tos)-Pro-Hyp-Gly-Thia-Ser-D-Tic-Oic-Arg-OH
MS(FAB) : 1458 (M + H)

**Beispiel 122:**

H-Lys-Lys(-CO-NH-$C_6H_5$)-Pro-Hyp-Gly-Thia-Ser-D-Tic-Oic-Arg-OH
MS(FAB) : 1367 (M + H)

**Beispiel 123:**

H-Lys-Lys(Nicotinoyl)-Pro-Hyp-Gly-Thia-Ser-D-Tic-Oic-Arg-OH
MS(FAB) : 1353 (M + H)

**Beispiel 124:**

H-Lys-Arg(Tos)-Pro-Hyp-Gly-Thia-Ser-D-Tic-Oic-Arg-OH
MS(FAB) : 1430 (M + H)

**Beispiel 125:**

H-D-Arg-Lys(Nicotinoyl)-Pro-Pro-Gly-Phe-Ser-D-Tic-Oic-Arg-OH
MS(FAB) : 1359 (M + H)

**Beispiel 126:**

H-D-Arg-Lys(-CO-NH-$C_6H_5$)-Pro-Pro-Gly-Phe-Ser-D-Tic-Oic-Arg-OH
MS(FAB) : 1373 (M + H)

**Beispiel 127:**

H-D-Arg-Arg(Tos)-Pro-Pro-Gly-Phe-Ser-D-Tic-Oic-Arg-OH
MS(FAB) : 1436 (M + H)

**Beispiel 128:**

H-Lys-Lys(Nicotinoyl)-Pro-Pro-Gly-Phe-Ser-D-Tic-Oic-Arg-OH
MS(FAB) : 1331 (M + H)

**Beispiel 129:**

H-Lys-Lys(-CO-NH-$C_6H_5$)-Pro-Pro-Gly-Phe-Ser-D-Tic-Oic-Arg-OH
MS(FAB) : 1345 (M + H)

**Beispiel 130:**

H-Lys-Arg(Tos)-Pro-Pro-Gly-Phe-Ser-D-Tic-Oic-Arg-OH
MS(FAB) : 1408 (M + H)

**Beispiel 131:**

H-D-Arg-Lys(Nicotinoyl)-Pro-Hyp-Gly-Phe-Ser-D-Tic-Oic-Arg-OH
MS(FAB) : 1375 (M + H)

**Beispiel 132:**

H-D-Arg-Lys(-CO-NH-$C_6H_5$)-Pro-Hyp-Gly-Phe-Ser-D-Tic-Oic-Arg-OH
MS(FAB) : 1389 (M + H)

**Beispiel 133:**

H-D-Arg-Arg(Tos)-Pro-Hyp-Gly-Phe-Ser-D-Tic-Oic-Arg-OH
MS(FAB) : 1452 (M + H)

**Beispiel 134:**

H-Lys-Lys(Nicotinoyl)-Pro-Hyp-Gly-Phe-Ser-D-Tic-Oic-Arg-OH
MS(FAB) : 1347 (M + H)

**Beispiel 135:**

H-Lys-Lys(-CO-NH-$C_6H_5$)-Pro-Hyp-Gly-Phe-Ser-D-Tic-Oic-Arg-OH
MS(FAB) : 1361 (M + H)

**Beispiel 136:**

H-Lys-Arg(Tos)-Pro-Hyp-Gly-Phe-Ser-D-Tic-Oic-Arg-OH
MS(FAB) : 1424 (M + H)

**Beispiel 137:**

H-D-Arg-Orn(Nicotinoyl)-Pro-Pro-Gly-Thia-Ser-D-Tic-Oic-Arg-OH
MS(FAB) : 1351 (M + H)

**Beispiel 138:**

H-D-Arg-Orn(-CO-NH-$C_6H_5$)-Pro-Pro-Gly-Thia-Ser-D-Tic-Oic-Arg-OH
MS(FAB) : 1428 (M + H)

**Beispiel 139:**

H-Lys-Orn(Nicotinoyl)-Pro-Pro-Gly-Thia-Ser-D-Tic-Oic-Arg-OH
MS(FAB) : 1323 (M + H)

**Beispiel 140:**

H-Lys-Orn(-CO-NH-$C_6H_5$)-Pro-Pro-Gly-Thia-Ser-D-Tic-Oic-Arg-OH
MS(FAB) : 1337 (M + H)

**Beispiel 141:**

H-D-Arg-Orn(Nicotinoyl)-Pro-Hyp-Gly-Thia-Ser-D-Tic-Oic-Arg-OH
MS(FAB) : 1367 (M + H)

**Beispiel 142:**

H-D-Arg-Orn(-CO-NH-$C_6H_5$)-Pro-Hyp-Gly-Thia-Ser-D-Tic-Oic-Arg-OH
MS(FAB) : 1381 (M + H)

**Beispiel 143:**

H-Lys-Orn(Nicotinoyl)-Pro-Hyp-Gly-Thia-Ser-D-Tic-Oic-Arg-OH
MS(FAB) : 1339 (M + H)

**Beispiel 144:**

H-Lys-Orn(-CO-NH-$C_6H_5$)-Pro-Hyp-Gly-Thia-Ser-D-Tic-Oic-Arg-OH
MS(FAB) : 1353 (M + H)

**Beispiel 145:**

H-D-Arg-Orn(Nicotinoyl)-Pro-Pro-Gly-Phe-Ser-D-Tic-Oic-Arg-OH
MS(FAB) : 1345 (M + H)

**Beispiel 146:**

H-D-Arg-Orn(-CO-NH-$C_6H_5$)-Pro-Pro-Gly-Phe-Ser-D-Tic-Oic-Arg-OH
MS(FAB) : 1359 (M + H)

**Beispiel 147:**

H-Lys-Orn(Nicotinoyl)-Pro-Pro-Gly-Phe-Ser-D-Tic-Oic-Arg-OH
MS(FAB) : 1317 (M + H)

**Beispiel 148:**

H-Lys-Orn(-CO-NH-$C_6H_5$)-Pro-Pro-Gly-Phe-Ser-D-Tic-Oic-Arg-OH
MS(FAB) : 1331 (M + H)

**Beispiel 149:**

H-D-Arg-Orn(Nicotinoyl)-Pro-Hyp-Gly-Phe-Ser-D-Tic-Oic-Arg-OH
MS(FAB) : 1361 (M + H)

**Beispiel 150:**

H-D-Arg-Orn(CO-NH-$C_6H_5$)-Pro-Hyp-Gly-Phe-Ser-D-Tic-Oic-Arg-OH
MS(FAB) : 1375 (M + H)

**Beispiel 151:**

H-Lys-Orn(Nicotinoyl)-Pro-Hyp-Gly-Phe-Ser-D-Tic-Oic-Arg-OH
MS(FAB) : 1333 (M + H)

**Beispiel 152:**

H-Lys-Orn(-CO-NH-$C_6H_5$)-Pro-Hyp-Gly-Phe-Ser-D-Tic-Oic-Arg-OH
MS(FAB) : 1347 (M + H)

**Beispiel 153:**

H-Lys-Lys-Pro-Pro-Gly-Thia-Ser-(D)-Tic-Aoc-Arg-OH
MS(FAB) : 1218 (M + H)

**Beispiel 154:**

H-Lys-Lys-Pro-Hyp-Gly-Thia-Ser-(D)-Tic-Aoc-Arg-OH
MS(FAB) : 1234 (M + H)

**Beispiel 155:**

H-Lys-Lys-Hyp-Pro-Gly-Thia-Ser-(D)-Tic-Aoc-Arg-OH
MS(FAB) : 1234 (M + H)

**Beispiel 156:**

H-Lys-Lys-Pro-Pro-Gly-Phe-Ser-(D)-Tic-Aoc-Arg-OH
MS(FAB) : 1212 (M + H)

**Beispiel 157:**

H-Lys-Lys-Pro-Hyp-Gly-Phe-Ser-(D)-Tic-Aoc-Arg-OH
MS(FAB) : 1228 (M + H)

**Beispiel 158:**

H-Lys-Lys-Pro-Pro-Gly-Thia-Ser-(D)-Tic-Oic-Arg-OH
MS(FAB) : 1232 (M + H)

**Beispiel 159:**

H-Lys-Lys-Pro-Hyp-Gly-Thia-Ser-(D)-Tic-Oic-Arg-OH
MS(FAB) : 1248 (M + H)

**Beispiel 160:**

H-Lys-Lys-Hyp-Pro-Gly-Thia-Ser-(D)-Tic-Oic-Arg-OH
MS(FAB) : 1226 (M + H)

**Beispiel 161:**

H-Lys-Lys-Pro-Hyp-Gly-Phe-Ser-(D)-Tic-Oic-Arg-OH
MS(FAB) : 1242 (M + H)

Die Beispiele 162 - 164 wurden analog Beispiel 32 unter Verwendung des in der EP-A 322348 beschriebenen Harzes mit der Struktur

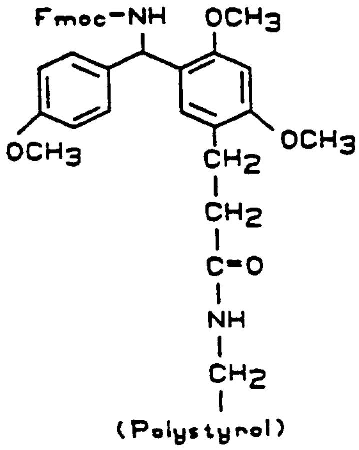

hergestellt.

**Beispiel 162:**

H-D-Arg-Arg-Pro-Hyp-Gly-Phe-Ser-D-Tic-Aoc-Arg-$NH_2$
MS(FAB) : 1283 (M + H)

**Beispiel 163:**

H-D-Arg-ARg-Hyp-Pro-Gly-Phe-Ser-D-Tic-Aoc-Arg-$NH_2$
MS(FAB): 1283 (M + H)

**Beispiel 164:**

H-D-Arg-Arg-Pro-Pro-Gly-Phe-Ser-D-Tic-Aoc-Arg-$NH_2$
MS(FAB): 1267 (M + H)

## Patentansprüche
## Patentansprüche für folgende Vertragsstaaten : AT, BE, CH, DE, FR, GB, IT, LI, LU, NL, SE

**1.** Peptid der Formel I

A-B-C-E-F-K-(D)-Tic-G-M-F'-I (I),

in welcher bedeuten:

A

$a_1$) Wasserstoff, ($C_1$-$C_8$)-Alkyl, ($C_1$-$C_8$)-Alkanoyl, ($C_1$-$C_8$)-Alkoxycarbonyl oder ($C_1$-$C_8$)-Alkylsulfonyl,

in welchen jeweils 1, 2 oder 3 Wasserstoffatome gegebenenfalls durch 1, 2 oder drei gleiche oder verschiedene Reste aus der Reihe Carboxy, Amino, ($C_1$-$C_4$)-Alkyl, ($C_1$-$C_4$)-Alkylamino, Hydroxy, ($C_1$-$C_3$)-Alkoxy, Halogen, Di-($C_1$-$C_4$)-alkylamino, Carbamoyl, Suffamoyl, ($C_1$-$C_4$)-Alkoxycarbonyl, ($C_6$-$C_{12}$)-Aryl und ($C_6$-$C_{12}$)-Aryl-($C_1$-$C_5$)-alkyl ersetzt sind,

oder in welchen jeweils 1 Wasserstoffatom gegebenenfalls durch einen Rest aus der Reihe ($C_3$-$C_8$)-6ycloalkyl, ($C_1$-$C_4$)-Alkylsulfonyl, ($C_1$-$C_4$)-Alkylsulfinyl, ($C_6$-$C_{12}$)-Aryl-($C_1$-$C_4$)-alkylsulfonyl, ($C_6$-$C_{12}$)-Aryl-($C_1$-$C_4$)-alkylsulfinyl, ($C_6$-$C_{12}$)-Aryloxy, ($C_3$-$C_9$)-Heteroaryl und ($C_3$-$C_9$)-Heteroaryloxy

und 1 oder 2 Wasserstoffatome durch 1 oder 2 gleiche oder verschiedene Reste aus der Reihe Carboxy, Amino, ($C_1$-$C_4$)-Alkylamino, Hydroxy, ($C_1$-$C_4$)-Alkoxy, Halogen, Di-($C_1$-$C_4$)-alkylamino, Carbamoyl, Sulfamoyl, ($C_1$-$C_4$)-Alkyloxycarbonyl, ($C_6$-$C_{12}$)-Aryl und ($C_6$-$C_{12}$)-Aryl-($C_1$-$C_5$)-alkyl ersetzt sind,

$a_2$) ($C_3$-$C_8$)-Cycloalkyl, Carbamoyl,

das gegebenenfalls am Stickstoff durch ($C_1$-$C_6$)-Alkyl oder ($C_6$-$C_{12}$)-Aryl substituiert sein kann,

($C_6$-$C_{12}$)-Aryl, ($C_7$-$C_{18}$)-Aryloyl, ($C_6$-$C_{12}$)-Arylsulfonyl oder ($C_3$-$C_9$)-Heteroaryl oder ($C_3$-$C_9$)-Heteroaryloyl, wobei in den unter $a_1$) und $a_2$) definierten Resten jeweils Heteroaryl, Aryloyl, Arylsulfonyl und Heteroaryloyl gegebenenfalls durch 1, 2, 3 oder 4 verschiedene Reste aus der Reihe Carboxy, Amino, Nitro, ($C_1$-$C_4$)-Alkylamino, Hydroxy, ($C_1$-$C_4$)-Alkyl, ($C_1$-$C_4$)-Alkoxy, Halogen, Cyano, Di-($C_1$-$C_4$)-alkylamino, Carbamoyl, Sulfamoyl und ($C_1$-$C_4$)-Alkoxycarbonyl substituiert ist, oder

$a_3$) einen Rest der Formel II,

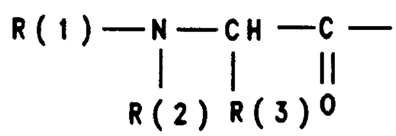 II

mit

R(1) wie A unter $a_1$) oder $a_2$) definiert,

R(2) Wasserstoff oder Methyl,

R(3) Wasserstoff oder ($C_1$-$C_6$)-Alkyl, vorzugsweise ($C_1$-$C_4$)-Alkyl, das gegebenenfalls durch Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstitiuert ist,

wobei substituiertes Amino für eine Verbindung -NH-A- und substituiertes Guanidino für eine Verbindung -NH-C(NH)-NH-A stehen, in denen A wie unter $a_1$) oder $a_2$) definiert ist;

B Arg, Lys, Orn, 2,4-Diaminobutyroyl oder einen L-Homoargininrest,

wobei jeweils die Amino- bzw. die Guanidinogruppe der Seitenkette durch A wie unter $a_1$) oder $a_2$) beschrieben substituiert sein kann;

C eine Verbindung der Formel IIIa oder IIIb

G'-G'-Gly (IIIa)

G'-NH-$(CH_2)_n$-CO (IIIb),

mit

G' unabhängig voneinander einem Rest der Formel IV

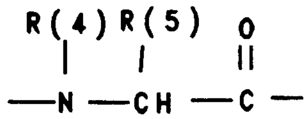

IV

worin R(4) und R(5) zusammen mit den diesen tragenden Atomen ein heterocyclisches mono-, bi- oder tricyclisches Ringsystem mit 2 bis 15 C-Atomen bilden,

und n 2 bis 8 ist;

E den Rest von Phenylalanin,

das gegebenenfalls durch Halogen in der 2-, 3- oder 4-Position substituiert ist, Tyrosin, O-Methyltyrosin, 2-Thienylalanin, 2-Pyridylalanin oder Naphthylalanin;

F unabhängig voneinander den Rest einer neutralen, sauren oder basischen, aliphatischen oder aromatischen Aminosäure,

die in der Seitenkette substituiert sein kann,

oder eine direkte Bindung;

(D)-Tic den Rest der Formel V

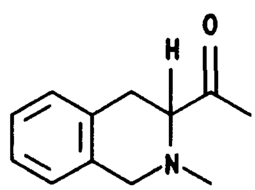

V

G wie G' oder eine direkte Bindung;

F' den Rest der basischen Aminosäuren Arg oder Lys in der L- oder D-Form oder eine direkte Bindung,

wobei die Guanidinogruppe bzw. Aminogruppe der Seitenkette durch A wie unter $a_1$) oder $a_2$) definiert substituiert sein kann,

oder einen Rest -NH-$(CH_2)_n$- mit n gleich 2 - 8,

oder eine direkte Bindung:

I -OH, $-NH_2$ oder $-NHC_2H_5$;

K den Rest -NH-$(CH_2)_x$-CO mit x gleich 1 - 4 oder eine direkte Bindung;

M wie F definiert,

sowie deren physiologisch verträgliche Salze.

**2.** Peptid der Formel I gemäß Anspruch 1, in welcher bedeuten:

B Arg, Orn oder Lys,

wobei die Guanidinogruppe bzw. die Aminogruppe der Seitenkette unsubstituiert ist oder durch ($C_1$-$C_8$)-Alkanoyl, ($C_7$-$C_{13}$)-Aryloyl, ($C_3$-$C_9$)-Heteroaryloyl, ($C_1$-$C_8$)-Alkylsuffonyl oder

($C_6$-$C_{12}$)-Arylsulfonyl substituiert sein kann, wobei die Aryl-, Heteroaryl-, Aryloyl-, Arylsulfonyl- und Heteroaryloylreste wie unter $a_2$) beschrieben mit gegebenenfalls 1, 2, 3 oder 4 gleichen oder verschiedenen Resten substituiert sein können;

E Phenylalanin, 2-Chlorphenylalanin, 3-Chlorphenylalanin, 4- Chlorphenylalanin, 2-Fluorphenylalanin, 3-Fluorphenylalanin, 4-Fluorphenylalanin, Tyrosin, O-Methyltyrosin oder $\beta$-(2-Thienyl)-alanin;

K eine direkte Bindung;

M eine direkte Bindung.

**3.** Peptid der Formel I nach Anspruch 1, worin bedeuten:

A Wasserstoff, (D)- oder (L)-H-Arg, (D)- oder (L)-H-Lys oder (D)- oder (L)-H-Orn;

B Arg, Orn oder Lys,
wobei die Guanidinogruppe bzw. die Aminogruppe der Seitenkette durch Wasserstoff, ($C_1$-$C_8$)-Alkanoyl, ($C_7$-$C_{13}$)-Aryloyl, ($C_3$-$C_9$)-Heteroaryloyl, ($C_1$-$C_8$)-Alkylsulfonyl oder ($C_6$-$C_{12}$)-Arylsulfonyl substituiert sein kann,
wobei die Aryl, Heteroaryl-, Aryloyl-, Arylsulfonyl- und Heteroaryloylreste gegebenenfalls mit 1, 2, 3 oder 4 gleichen oder verschiedenen Resten aus der Reihe Methyl, Methoxy und Halogen substituiert sein können;

C Pro-Pro-Gly, Hyp-Pro-Gly oder Pro-Hyp-Gly;

E Phe oder Thia;

F Ser, Hser, Lys, Leu, Val, Nle, Ile oder Thr;

K eine direkte Bindung;

M eine direkte Bindung;

G den Rest eines heterocyclischen Ringsystems der Formel IV, ausgewählt aus den Resten der Heterocyclen Pyrrolidin (A), Piperidin (B), Tetrahydroisochinolin (C), cis- oder trans-Decahydroisochinolin (D), cis-endo-Octahydroindol (E), cis-exo-Octahydroindol (E), trans-Octahydroindol (E), cis-endo-, cis-exo-, trans-Octahydrocyclopentano[b]pyrrol (F), oder Hydroxyprolin (V);

F' Arg;

I OH.

**4.** Peptid der Formel I nach Anspruch 1, dadurch gekennzeichnet, daß es ausgewählt ist aus der Gruppe:
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH,
H-(D)-Arg-Arg-Hyp-Pro-Gly-Phe-Ser-(D)-Tic-Oic-Arg-OH,
H-(D)-Arg-Arg-Pro-Pro-Gly-Phe-Ser-(D)-Tic-Oic-Arg-OH.

**5.** Verfahren zur Herstellung eines Peptids der Formel I gemäß einem oder mehreren der Ansprüche 1 bis 4, dadurch gekennzeichnet, daß man
a) ein Fragment mit C-terminaler freier Carboxylgruppe oder dessen aktiviertes Derivat mit einem entsprechenden Fragment mit N-terminaler freier Aminogruppe umsetzt oder
b) das Peptid stufenweise aufbaut,
in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der Formel I gegebenenfalls in ihr physiologisch verträgliches Salz überführt.

**6.** Verwendung eines Peptids der Formel I gemäß einem der Ansprüche 1 - 4 zur Herstellung eines Medikaments zur Behandlung pathologischer Zustände, die durch Bradykinin und Bradykinin verwandte Peptide vermittelt, ausgelöst oder unterstützt werden.

**7.** Pharmazeutisches Mittel, enthaltend ein Peptid der Formel I gemäß einem oder mehreren der Ansprüche 1 bis 4.

**Patentansprüche für folgende Vertragsstaaten : ES, GR**

**1.** Verfahren zur Herstellung eines Peptids der Formel I

A-B-C-E-F-K-(D)-Tic-G-M-F'-I (I),

in welcher bedeuten:

A

$a_1$) Wasserstoff, $(C_1-C_8)$-Alkyl, $(C_1-C_8)$-Alkanoyl, $(C_1-C_8)$-Alkoxycarbonyl oder $(C_1-C_8)$-Alkylsulfonyl,

in welchen jeweils 1, 2 oder 3 Wasserstoffatome gegebenenfalls durch 1, 2 oder drei gleiche oder verschiedene Reste aus der Reihe Carboxy, Amino, $(C_1-C_4)$-Alkyl, $(C_1-C_4)$-Alkylamino, Hydroxy, $(C_1-C_3)$-Alkoxy, Halogen, Di-$(C_1-C_4)$-alkylamino, Carbamoyl, Sulfamoyl, $(C_1-C_4)$-Alkoxycarbonyl, $(C_6-C_{12})$-Aryl und $(C_6-C_{12})$-Aryl-$(C_1-C_5)$-alkyl ersetzt sind,

oder in welchen jeweils 1 Wasserstoffatom gegebenenfalls durch einen Rest aus der Reihe $(C_3-C_8)$-Cycloalkyl, $(C_1-C_4)$-Alkylsulfonyl, $(C_1-C_4)$-Alkylsulfinyl, $(C_6-C_{12})$-Aryl-$(C_1-C_4)$-alkylsulfonyl, $(C_6-C_{12})$-Aryl-$(C_1-C_4)$-alkylsulfinyl, $(C_6-C_{12})$-Aryloxy, $(C_3-C_9)$-Heteroaryl und $(C_3-C_9)$-Heteroaryloxy

und 1 oder 2 Wasserstoffatome durch 1 oder 2 gleiche oder verschiedene Reste aus der Reihe Carboxy, Amino, $(C_1-C_4)$-Alkylamino, Hydroxy, $(C_1-C_4)$-Alkoxy, Halogen, Di-$(C_1-C_4)$-alkylamino, Carbamoyl, Sulfamoyl, $(C_1-C_4)$-Alkyloxycarbonyl, $(C_6-C_{12})$-Aryl und $(C_6-C_{12})$-Aryl-$(C_1-C_5)$-alkyl ersetzt sind,

$a_2$) $(C_3-C_8)$-Cycloalkyl, Carbamoyl,

das gegebenenfalls am Stickstoff durch $(C_1-C_6)$-Alkyl oder $(C_6-C_{12})$-Aryl substituiert sein kann,

$(C_6-C_{12})$-Aryl, $(C_7-C_{18})$-Aryloyl, $(C_6-C_{12})$-Arylsulfonyl oder $(C_3-C_9)$-Heteroaryl oder $(C_3-C_9)$-Heteroaryloyl,

wobei in den unter $a_1$) und $a_2$) definierten Resten jeweils Heteroaryl, Aryloyl, Arylsulfonyl und Heteroaryloyl gegebenenfalls durch 1, 2, 3 oder 4 verschiedene Reste aus der Reihe Carboxy, Amino, Nitro, $(C_1-C_4)$-Alkylamino, Hydroxy, $(C_1-C_4)$-Alkyl, $(C_1-C_4)$-Alkoxy, Halogen, Cyano, Di-$(C_1-C_4)$-alkylamino, Carbamoyl, Sulfamoyl und $(C_1-C_4)$-Alkoxycarbonyl substituiert ist, oder

$a_3$) einen Rest der Formel II,

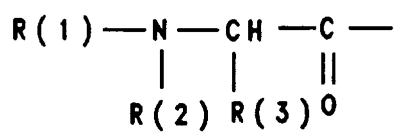

II

mit

R(1) wie A unter $a_1$) oder $a_2$) definiert,

R(2) Wasserstoff oder Methyl,

R(3) Wasserstoff oder $(C_1-C_6)$-Alkyl, vorzugsweise $(C_1-C_4)$-Alkyl, das gegebenenfalls durch Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cycloheryl monosubstituiert ist,

wobei substituiertes Amino für eine Verbindung -NH-A- und substituiertes Guanidino für eine Verbindung -NH-C(NH)-NH-A stehen, in denen A wie unter $a_1$) oder $a_2$) definiert ist;

B Arg, Lys, Orn, 2,4-Diaminobutyroyl oder einen L-Homoargininrest,

wobei jeweils die Amino- bzw. die Guanidinogruppe der Sertenkette durch A wie unter $a_1$) oder $a_2$) beschrieben substituiert sein kann;

C eine Verbindung der Formel IIIa oder IIIb

G'-G'-Gly (IIIa)

G'-NH-$(CH_2)_n$-CO (IIIb),

mit
G' unabhängig voneinander einem Rest der Formel IV

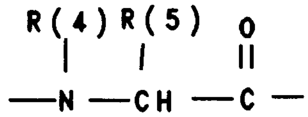

IV

worin R(4) und R(5) zusammen mit den diesen tragenden Atomen ein heterocyclisches mono-, bi- oder tricyclisches Ringsystem mit 2 bis 15 C-Atomen bilden,
und n 2 bis 8 ist;

E den Rest von Phenylalanin,
das gegebenenfalls durch Halogen in der 2-, 3- oder 4-Position substituiert ist, Tyrosin, O-Methyltyrosin, 2-Thienylalanin, 2-Pyridylalanin oder Naphthylalanin;

F unabhängig voneinander den Rest einer neutralen, sauren oder basischen, aliphatischen oder aromatischen Aminosäure,
die in der Seitenkette substituiert sein kann,
oder eine direkte Bindung;

(D)-Tic den Rest der Formel V

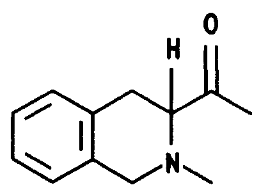

V

G wie G' oder eine direkte Bindung;

F' den Rest der basischen Aminosäuren Arg oder Lys in der L- oder D-Form oder eine direkte Bindung,
wobei die Guanidinogruppe bzw. Aminogruppe der Seitenkette durch A wie unter $a_1$) oder $a_2$) definiert substituiert sein kann,
oder einen Rest $-NH-(CH_2)_n-$ mit n gleich 2 - 8,
oder eine direkte Bindung:

I $-OH$, $-NH_2$ oder $-NHC_2H_5$;

K den Rest $-NH-(CH_2)_x-CO$ mit x gleich 1 - 4 oder eine direkte Bindung;

M wie F definiert,

sowie von deren physiologisch verträglichen Salzen,
dadurch gekennzeichnet, daß man

a) ein Fragment mit C-terminaler freier Carboxylgruppe oder dessen aktiviertes Derivat mit einem entsprechenden Fragment mit N-terminaler freier Aminogruppe umsetzt oder

b) das Peptid stufenweise aufbaut,

in der nach (a) oder (b) erhaltenen Verbindung gegebenenfalls eine oder mehrere zum Schutz anderer Funktionen temporär eingefuhrte Schutzgruppen abspaltet und die so erhaltenen Verbindungen der Formel I gegebenenfalls in ihr physiologisch verträgliches Salz überführt.

**2.** Verfahren nach Anspruch 1 zur Herstellung eines Peptids der Formel I,
in welcher bedeuten:

B Arg, Orn oder Lys,
wobei die Guanidinogruppe bzw. die Aminogruppe der Seitenkette unsubstituiert ist oder durch ($C_1$-$C_8$)-Alkanoyl, ($C_7$-$C_{13}$)-Aryloyl, ($C_3$-$C_9$)-Heteroaryloyl, ($C_1$-$C_8$)-Alkylsulfonyl oder ($C_6$-$C_{12}$)-Arylsulfonyl substituiert sein kann, wobei die Aryl-, Heteroaryl-, Aryloyl-, Arylsulfonyl- und Heteroaryloylreste wie unter $a_2$) beschrieben mit gegebenenfalls 1, 2, 3 oder 4 gleichen oder verschiedenen Resten substituiert sein können;

E Phenylalanin, 2-Chlorphenylalanin, 3-Chlorphenylalanin, 4- Chlorphenylalanin, 2-Fluorphenylalanin, 3-Fluorphenylalanin, 4-Fluorphenylalanin, Tyrosin, O-Methyltyrosin oder $\beta$-(2-Thienyl)-

alanin;
K eine direkte Bindung;
M eine direkte Bindung.

**3.** Verfahren nach Anspruch 1 zur Herstellung eines Peptids der Formel I, in welcher bedeuten:
A Wasserstoff, (D)- oder (L)-H-Arg, (D)- oder (L)-H-Lys oder (D)- oder (L)-H-Orn;
B Arg, Orn oder Lys,
wobei die Guanidinogruppe bzw. die Aminogruppe der Seitenkette durch Wasserstoff, ($C_1$-$C_8$)-Alkanoyl, ($C_7$-$C_{13}$)-Aryloyl, ($C_3$-$C_9$)-Heteroaryloyl, ($C_1$-$C_8$)-Alkylsulfonyl oder ($C_6$-$C_{12}$)-Arylsulfonyl substituiert sein kann,
wobei die Aryl, Heteroaryl-, Aryloyl-, Arylsulfonyl- und Heteroaryloylreste gegebenenfalls mit 1, 2, 3 oder 4 gleichen oder verschiedenen Resten aus der Reihe Methyl, Methoxy und Halogen substituiert sein können;
C Pro-Pro-Gly, Hyp-Pro-Gly oder Pro-Hyp-Gly;
E Phe oder Thia;
F Ser, Hser, Lys, Leu, Val, Nle, Ile oder Thr;
K eine direkte Bindung;
M eine direkte Bindung;
G den Rest eines heterocyclischen Ringsystems der Formel IV, ausgewählt aus den Resten der Heterocyclen Pyrrolidin (A), Piperidin (B), Tetrahydroisochinolin (C), cis- oder trans-Decahydroisochinolin (D), cis-endo-Octahydroindol (E), cis-exo-Octahydroindol (E), trans-Octahydroindol (E), cis-endo-, cis-exo-, trans-Octahydrocyclopentano[b]pyrrol (F), oder Hydroxyprolin (V);
F' Arg;
I OH.

**4.** Verfahren nach Anspruch 1 zur Herstellung eines Peptids der Formel I, in welchem ein Peptid aus der Gruppe hergestellt wird, die besteht aus:
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH,
H-(D)-Arg-Arg-Hyp-Pro-Gly-Phe-Ser-(D)-Tic-Oic-Arg-OH,
H-(D)-Arg-Arg-Pro-Pro-Gly-Phe-Ser-(D)-Tic-Oic-Arg-OH.

**5.** Verwendung eines gemäß einem der Ansprüche 1 - 4 hergestellten Peptids der Formel I zur Herstellung eines Medikaments zur Behandlung pathologischer Zustände, die durch Bradykinin und Bradykinin verwandte Peptide vermittelt, ausgelöst oder unterstützt werden.

**Claims**
**Claims for the following Contracting States : AT, BE, CH, DE, FR, GB, IT, LI, LU, NL, SE**

**1.** A peptide of the formula I

A-B-C-E-F-K-(D)-Tic-G-M-F'-I I,

in which
A
$a_1$) is hydrogen, ($C_1$-$C_8$)-alkyl, ($C_1$-$C_8$)-alkanoyl, ($C_1$-$C_8$)-alkoxycarbonyl or ($C_1$-$C_8$)-alkylsulfonyl,
in which in each case 1, 2 or 3 hydrogen atoms are optionally replaced by 1, 2 or 3 identical or different radicals from the group comprising carboxyl, amino, ($C_1$-$C_4$)-alkyl, ($C_1$-$C_4$)-alkylamino, hydroxyl, ($C_1$-$C_3$)-alkoxy, halogen, di-($C_1$-$C_4$)-alkylamino, carbamoyl, sulfamoyl, ($C_1$-$C_4$)-alkoxycarbonyl, ($C_6$-$C_{12}$)-aryl and ($C_6$-$C_{12}$)-aryl-($C_1$-$C_5$)-alkyl,
or in which in each case 1 hydrogen atom is optionally replaced by a radical from the group comprising ($C_3$-$C_8$)-cycloalkyl, ($C_1$-$C_4$)-alkylsulfonyl, ($C_1$-$C_4$)-alkylsulfinyl, ($C_6$-$C_{12}$)-aryl-($C_1$-$C_4$)-alkylsulfonyl, ($C_6$-$C_{12}$)-aryl-($C_1$-$C_4$)-alkylsulfinyl, ($C_6$-$C_{12}$)-aryloxy,

($C_3$-$C_9$)-heteroaryl and ($C_3$-$C_9$)-heteroaryloxy

and

1 or 2 hydrogen atoms are replaced by 1 or 2 identical or different radicals from the group comprising carboxyl, amino, ($C_1$-$C_4$)-alkylamino, hydroxyl, ($C_1$-$C_4$)-alkoxy, halogen, di-($C_1$-$C_4$)-alkylamino, carbamoyl, sulfamoyl, ($C_1$-$C_4$)-alkyloxycarbonyl, ($C_6$-$C_{12}$)-aryl and ($C_6$-$C_{12}$)-aryl- ($C_1$-$C_5$)-alkyl,

$a_2$) is ($C_3$-$C_8$)-cycloalkyl, carbamoyl,

which can optionally be substituted on the nitrogen by ($C_1$-$C_6$)-alkyl or ($C_6$-$C_{12}$)-aryl,

($C_6$-$C_{12}$)-aryl, ($C_7$-$C_{18}$)-aryloyl, ($C_6$-$C_{12}$)-arylsulfonyl or ($C_3$-$C_9$)-heteroaryl or ($C_3$-$C_9$)-heteroaryloyl,

where in the radicals defined under $a_1$) and $a_2$) in each case heteroaryl, aryloyl, arylsulfonyl and heteroaryloyl is optionally substituted by 1, 2, 3 or 4 different radicals from the group comprising carboxyl, amino, nitro, ($C_1$-$C_4$)-alkylamino, hydroxyl, ($C_1$-$C_4$)-alkyl, ($C_1$-$C_4$)-alkoxy, halogen, cyano, di-($C_1$-$C_4$)-alkylamino, carbamoyl, sulfamoyl and ($C_1$-$C_4$)-alkoxycarbonyl, or

$a_3$) is a radical of the formula II

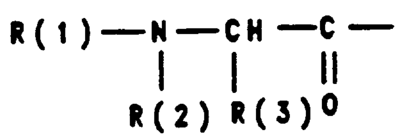

II,

where

| | |
|---|---|
| R(1) | is defined as A under $a_1$) or $a_2$), |
| R(2) | is hydrogen or methyl, |
| R(3) | is hydrogen or ($C_1$-$C_6$)-alkyl, preferably ($C_1$-$C_4$)-alkyl, which is optionally monosubstituted by amino, substituted amino, hydroxyl, carboxyl, carbamoyl, guanidino, substituted guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pyridyl or cyclohexyl, where substituted amino is a compound -NH-A- and substituted guanidino is a compound -NH-C(NH)-NH-A, in which A is defined as under $a_1$) or $a_2$); |
| B | is Arg, Lys, Orn, 2,4-diaminobutyroyl or an L-homoarginine radical, where in each case the amino or the guanidino group of the side chain can be substituted by A as described under $a_1$) or $a_2$); |
| C | is a compound of the formula IIIa or IIIb |

G'-G'-Gly (IIIa)

G'-NH-$(CH_2)_n$-CO (IIIb)

where

G' independently of one another are a radical of the formula IV

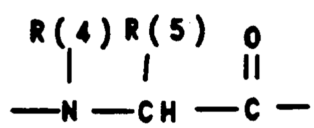

IV,

in which

R(4) and R(5) together with the atoms carrying them form a heterocyclic mono-, bi- or tricyclic ring system having 2 to 15 carbon atoms, and

n is 2 to 8;

E is the radical of phenylalanine,

which is optionally substituted by halogen in the 2-, 3- or 4-position,

tyrosine, O-methyltyrosine, 2-thienylalanine, 2-pyridylalanine or naphthylalanine;

F independently of one another is the radical of a neutral, acidic or basic, aliphatic or aromatic amino acid, which can be substituted in the side chain, or is a direct bond;

(D)-Tic is the radical of the formula V

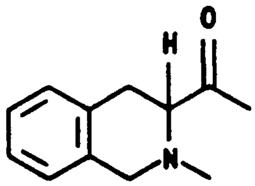

V

G is as defined for G' or is a direct bond;

F' is the radical of the basic amino acids Arg or Lys, in the L- or D-form, or a direct bond, where the guanidino group or amino group of the side chain can be substituted by A as described under $a_1$) or $a_2$), or a radical $-NH-(CH_2)_n-$ with n = 2 - 8;

I is -OH, $-NH_2$ or $-NHC_2H_5$;

K is the radical $-NH-(CH_2)_x-CO$ with x = 1 - 4 or is a direct bond;

M is as defined for F;

or its physiologically tolerable salts.

**2.** A peptide of the formula I as claimed in claim 1, in which

B is Arg, Orn or Lys, where the guanidino group or the amino group of the side chain is unsubstituted or can be substituted by $(C_1-C_8)$-alkanoyl, $(C_7-C_{13})$-aryloyl, $(C_3-C_9)$-heteroaryloyl, $(C_1-C_8)$-alkylsulfonyl or $(C_6-C_{12})$-arylsulfonyl, where the aryl, heteroaryl, aryloyl, arylsulfonyl and heteroaryloyl radicals can be substituted as described under $a_2$) by optionally 1, 2, 3 or 4 identical or different radicals;

E is phenylalanine, 2-chlorophenylalanine, 3-chlorophenylalanine, 4-chlorophenylalanine, 2-fluorophenylalanine, 3-fluorophenylalanine, 4-fluorophenylalanine, tyrosine, O-methyltyrosine or $\beta$-(2-thienyl)alanine;

K is a direct bond;

M is a direct bond.

**3.** A peptide of the formula I as claimed in claim 1, in which

A is hydrogen, (D)- or (L)-H-Arg, (D)- or (L)-H-Lys or (D)- or (L)-H-Orn;

B is Arg, Orn or Lys, where the guanidino group or the amino group of the side chain can be substituted by hydrogen, $(C_1-C_8)$-alkanoyl, $(C_7-C_{13})$-aryloyl, $(C_3-C_9)$-heteroaryloyl, $(C_1-C_8)$-alkylsulfonyl or $(C_6-C_{12})$-arylsulfonyl,

where the aryl, heteroaryl, aryloyl, arylsulfonyl and heteroaryloyl radicals can optionally be substituted by 1, 2, 3 or 4 identical or different radicals from the group comprising methyl, methoxy and halogen;

C is Pro-Pro-Gly, Hyp-Pro-Gly or Pro-Hyp-Gly;

E is Phe or Thia;

F is Ser, Hser, Lys, Leu, Val, Nle, Ile or Thr;

K is a direct bond;

M is a direct bond;

G is the radical of a heterocyclic ring system of the formula IV, selected from the radicals of the heterocycles pyrrolidine (A), piperidine (B), tetrahydroisoquinoline (C), cis- or trans-decahydroisoquinoline (D), cis-endooctahydroindole (E), cis-exo-octahydroindole (E), trans-octahydroindole (E), cis-endo-, cis-exo- or trans-octahydrocyclopentano[b]pyrrole (F), or hydroxyproline (V);

F' is Arg;

I is OH.

4. A peptide of the formula I as claimed in claim 1, which is selected from the group comprising:
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH,
H-(D)-Arg-Arg-Hyp-Pro-Gly-Phe-Ser-(D)-Tic-Oic-Arg-OH,
H-(D)-Arg-Arg-Pro-Pro-Gly-Phe-Ser-(D)-Tic-Oic-Arg-OH.

5. A process for the preparation of a peptide of the formula I as claimed in one or more of claims 1 to 4, which comprises
a) reacting a fragment having a C-terminal free carboxyl group or its activated derivative with an appropriate fragment having an N-terminal free amino group or
b) synthesizing the peptide stepwise,
optionally splitting off one or more protective groups temporarily introduced for the protection of other functions in the compound obtained according to (a) or (b) and optionally converting the compounds of the formula I thus obtained into their physiologically tolerable salts.

6. Use of a peptide of the formula I as claimed in one of claims 1 to 4 for the preparation of a medicament for the treatment of pathological states which are mediated, caused or supported by bradykinin and bradykinin-related peptides.

7. A pharmaceutical agent containing a peptide of the formula I as claimed in one or more of claims 1 to 4.

**Claims for the following Contracting States : ES, GR**

1. A process for the preparation of a peptide of the formula I

A-B-C-E-F-K-(D)-Tic-G-M-F'-I I,

in which
A
$a_1$) is hydrogen, ($C_1$-$C_8$)-alkyl, ($C_1$-$C_8$)-alkanoyl, ($C_1$-$C_8$) -alkoxycarbonyl or ($C_1$-$C_8$)-alkylsulfonyl,
in which in each case 1, 2 or 3 hydrogen atoms are optionally replaced by 1, 2 or 3 identical or different radicals from the group comprising carboxyl, amino, ($C_1$-$C_4$)-alkyl, ($C_1$-$C_4$)-alkylamino, hydroxyl, ($C_1$-$C_3$)-alkoxy, halogen, di-($C_1$-$C_4$)-alkylamino, carbamoyl, sulfamoyl, ($C_1$-$C_4$)-alkoxycarbonyl, ($C_6$-$C_{12}$)-aryl and ($C_6$-$C_{12}$)-aryl-($C_1$-$C_5$)-alkyl,
or in which in each case 1 hydrogen atom is optionally replaced by a radical from the group comprising ($C_3$-$C_8$)-cycloalkyl, ($C_1$-$C_4$)-alkylsulfonyl, ($C_1$-$C_4$)-alkylsulfinyl, ($C_6$-$C_{12}$)-aryl-($C_1$-$C_4$)-alkylsulfonyl, ($C_6$-$C_{12}$)-aryl-($C_1$-$C_4$)-alkylsulfinyl, ($C_6$-$C_{12}$)-aryloxy, ($C_3$-$C_9$)-heteroaryl and ($C_3$-$C_9$)-heteroaryloxy
and
1 or 2 hydrogen atoms are replaced by 1 or 2 identical or different radicals from the group comprising carboxyl, amino, ($C_1$-$C_4$)-alkylamino, hydroxyl, ($C_1$-$C_4$)-alkoxy, halogen, di-($C_1$-$C_4$)-alkylamino, carbamoyl, sulfamoyl, ($C_1$-$C_4$)-alkyloxycarbonyl, ($C_6$-$C_{12}$)-aryl and ($C_6$-$C_{12}$)-aryl-($C_1$-$C_5$)-alkyl,
$a_2$) is ($C_3$-$C_8$)-cycloalkyl, carbamoyl,
which can optionally be substituted on the nitrogen by ($C_1$-$C_6$)-alkyl or ($C_6$-$C_{12}$)-aryl,
($C_6$-$C_{12}$)-aryl, ($C_7$-$C_{18}$)-aryloyl, ($C_6$-$C_{12}$)-arylsulfonyl or ($C_3$-$C_9$)-heteroaryl or ($C_3$-$C_9$)-heteroaryloyl,
where in the radicals defined under $a_1$) and $a_2$) in each case heteroaryl, aryloyl, arylsulfonyl and heteroaryloyl is optionally substituted by 1, 2, 3 or 4 different radicals from the group comprising carboxyl, amino, nitro, ($C_1$-$C_4$)-alkylamino, hydroxyl, ($C_1$-$C_4$)-alkyl, ($C_1$-$C_4$)-alkoxy, halogen, cyano, di-($C_1$-$C_4$)-alkylamino, carbamoyl, sulfamoyl and

($C_1$-$C_4$)-alkoxycarbonyl, or

$a_3$) is a radical of the formula II

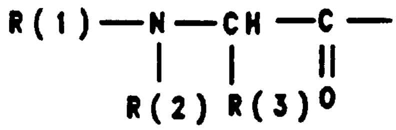

II,

where

R(1) is defined as A under $a_1$) or $a_2$),

R(2) is hydrogen or methyl,

R(3) is hydrogen or ($C_1$-$C_6$)-alkyl, preferably ($C_1$-$C_4$)-alkyl,
which is optionally monosubstituted by amino, substituted amino, hydroxyl, carboxyl, carbamoyl, guanidino, substituted guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pyridyl or cyclohexyl,
where substituted amino is a compound -NH-A- and substituted guanidino is a compound -NH-C(NH)-NH-A, in which A is defined as under $a_1$) or $a_2$);

B is Arg, Lys, Orn, 2,4-diaminobutyroyl or an L-homoarginine radical,
where in each case the amino or the guanidino group of the side chain can be substituted by A as described under $a_1$) or $a_2$);

C is a compound of the formula IIIa or IIIb

G'-G'-Gly (IIIa)

G'-NH-$(CH_2)_n$-CO (IIIb)

where

G' independently of one another are a radical of the formula IV

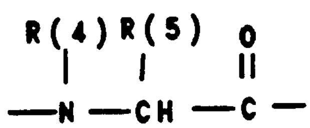

IV

in which

R(4) and R(5) together with the atoms carrying them form a heterocyclic mono-, bi- or tricyclic ring system having 2 to 15 carbon atoms, and

n is 2 to 8;

E is the radical of phenylalanine,
which is optionally substituted by halogen in the 2-, 3- or 4-position,
tyrosine, O-methyltyrosine, 2-thienylalanine, 2-pyridylalanine or naphthylalanine;

F independently of one another is the radical of a neutral, acidic or basic, aliphatic or aromatic amino acid,
which can be substituted in the side chain, or is a direct bond;

(D)-Tic is the radical of the formula V

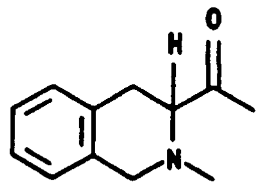

V

G is as defined for G' or is a direct bond;

F' is the radical of the basic amino acids Arg or Lys, in the L- or D-form, or a direct bond, where the guanidino group or amino group of the side chain can be substituted by A as described under $a_1$) or $a_2$), or a radical $-NH-(CH_2)_n-$ with n = 2 - 8;

I is -OH, $-NH_2$ or $-NHC_2H_5$;

K is the radical $-NH-(CH_2)_x-CO-$ with x = 1 - 4 or is a direct bond;

M is as defined for F;

or its physiologically tolerable salts,
which comprises

a) reacting a fragment having a C-terminal free carboxyl group or its activated derivative with an appropriate fragment having an N-terminal free amino group or

b) synthesizing the peptide stepwise,

optionally splitting off one or more protective groups temporarily introduced for the protection of other functions in the compound obtained according to (a) or (b) and optionally converting the compounds of the formula I thus obtained into their physiologically tolerable salts.

**2.** A process as claimed in claim 1 for the preparation of a peptide of the formula I, in which

B is Arg, Orn or Lys, where the guanidino group or the amino group of the side chain is unsubstituted or can be substituted by $(C_1-C_8)$-alkanoyl, $(C_7-C_{13})$-aryloyl, $(C_3-C_9)$-heteroaryloyl, $(C_1-C_8)$-alkylsulfonyl or $(C_6-C_{12})$-arylsulfonyl, where the aryl, heteroaryl, aryloyl, arylsulfonyl and heteroaryloyl radicals can be substituted as described under $a_2$) by optionally 1, 2, 3 or 4 identical or different radicals;

E is phenylalanine, 2-chlorophenylalanine, 3-chlorophenylalanine, 4-chlorophenylalanine, 2-fluorophenylalanine, 3-fluorophenylalanine, 4-fluorophenylalanine, tyrosine, O-methyltyrosine or $\beta$-(2-thienyl)alanine;

K is a direct bond;

M is a direct bond.

**3.** A process as claimed in claim 1 for the preparation of a peptide of the formula I, in which

A is hydrogen, (D)- or (L)-H-Arg, (D)- or (L)-H-Lys or (D)- or (L)-H-Orn;

B is Arg, Orn or Lys, where the guanidino group or the amino group of the side chain can be substituted by hydrogen, $(C_1-C_8)$-alkanoyl, $(C_7-C_{13})$-aryloyl, $(C_3-C_9)$-heteroaryloyl, $(C_1-C_8)$-alkylsulfonyl or $(C_6-C_{12})$-arylsulfonyl, where the aryl, heteroaryl, aryloyl, arylsulfonyl and heteroaryloyl radicals can optionally be substituted by 1, 2, 3 or 4 identical or different radicals from the group comprising methyl, methoxy and halogen;

C is Pro-Pro-Gly, Hyp-Pro-Gly or Pro-Hyp-Gly;

E is Phe or Thia;

F is Ser, Hser, Lys, Leu, Val, Nle, Ile or Thr;

K is a direct bond;

M is a direct bond;

G is the radical of a heterocyclic ring system of the formula IV, selected from the radicals of the heterocycles pyrrolidine (A), piperidine (B), tetrahydroisoquinoline (C), cis- or trans-decahydroisoquinoline (D), cis-endo-octahydroindole (E), cis-exo-octahydroindole (E), trans-octahydroindole (E), cis-endo-, cis-exo- or trans-octahydrocyclopentano[b]pyrrole (F), or hydroxyproline (V);

F' is Arg;
I is OH.

**4.** A process as claimed in claim 1 for the preparation of a peptide of the formula I, in which a peptide is prepared from the group which comprises:
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH,
H-(D)-Arg-Arg-Hyp-Pro-Gly-Phe-Ser-(D)-Tic-Oic-Arg-OH,
H-(D)-Arg-Arg-Pro-Pro-Gly-Phe-Ser-(D)-Tic-Oic-Arg-OH.

**5.** Use of a peptide of the formula I prepared as claimed in one of claims 1 to 4 for the preparation of a medicament for treatment of pathological states which are mediated, caused or supported by bradykinin and bradykinin-related peptides.

**Revendications**
**Revendications pour les Etats contractants suivants : AT, BE, CH, DE, FR, GB, IT, LI, LU, NL, SE**

**1.** Peptide de formule I

A-B-C-E-F-K-(D)-Tic-G-M-F'-I (I)

dans laquelle
A représente
$a_1$) un atome d'hydrogène ou un groupe alkyle en $C_1$-$C_8$, alcanoyle en $C_1$-$C_8$, alcoxy-($C_1$-$C_8$)-carbonyle ou alkyl($C_1$-$C_8$)-sulfonyle,
dans chacun desguels 1, 2 ou 3 atomes d'hydrogène sont éventuellement remplacés par 1, 2 ou 3 radicaux, identiques ou différents, choisis parmi des atomes d'halogène et des groupes carboxy, amlno, alkyle en $C_1$-$C_4$, alkyl($C_1$-$C_4$)-amino, hydroxy, alcoxy en $C_1$-$C_3$, dialkyl($C_1$-$C_4$)-amino, carbamoyle, sulfamoyle, alcoxy($C_1$-$C_4$)-carbonyle, aryle en $C_6$-$C_{12}$ et aryl($C_6$-$C_{12}$)-alkyle-($C_1$-$C_5$),
ou dans chacun desquels un atome d'hydrogène est éventuellement remplacé par un radical choisi parmi des radicaux cycloalkyle en $C_3$-$C_8$, alkyl($C_1$-$C_4$)-sulfonyle, alkyl($C_1$-$C_4$)-sulfinyle, aryl($C_6$-$C_{12}$)-alkyl($C_1$-$C_4$)-sulfonyle, aryl($C_6$-$C_{12}$)-alkyl($C_1$-$C_4$)-sulfinyle, aryloxy en $C_6$-$C_{12}$, hétéroaryle en $C_3$-$C_9$ et hétéroaryloxy en $C_3$-$C_9$,
et 1 ou 2 atomes d'hydrogène sont remplacés par 1 ou 2 radicaux, identiques ou différents, choisis parmi des atomes d'halogène et des groupes carboxy, amino, alkyl-($C_1$-$C_4$)amino, hydroxy, alcoxy en $C_1$-$C_4$, dialkyl(-$C_1$-$C_4$)-amino, carbamoyle, sulfamoyle, alkyloxy($C_1$-$C_4$)-carbonyle, aryle en $C_6$-$C_{12}$ et aryl($C_6$-$C_{12}$)-alkyle($C_1$-$C_5$),
$a_2$) un groupe cycloalkyle en $C_3$-$C_8$ ou carbamoyle,
qui peut éventuellement étre substitué à l'azote par un radical alkyle en $C_1$-$C_6$ ou aryle en $C_6$-$C_{12}$,
un groupe aryle en $C_6$-$C_{12}$, aryloyle en $C_7$-$C_{18}$, aryl-($C_6$-$C_{12}$)sulfonyle ou hétéroaryle en $C_3$-$C_9$ ou hétéroaryloyle en $C_3$-$C_9$,
dans les restes définis en $a_1$) et $a_2$), les radicaux hétéroaryle, aryloyle, arylsulfonyle et hétéroaryloyle étant éventuellement substitués chacun par 1, 2, 3 ou 4 radicaux différents choisis parmi des atomes d'halogène et des groupes carbory, amino, nitro, alkyl($C_1$-$C_4$)-amino, hydroxy, alkyle en $C_1$-$C_4$, alcoxy en $C_1$-$C_4$, cyano, dialkyl($C_1$-$C_4$)-amino, carbamoyle, sulfamoyle et alcoxy($C_1$-$C_4$)-carbonyle, ou
$a_3$) un radical de formule II

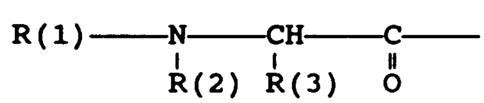 (II)

dans laquelle
R(1) est défini comme A en $a_1$) ou $a_2$),

R(2) représente un atome d'hydrogène ou le groupe méthyle,

R(3) représente un atome d'hydrogène ou un groupe alkyle en $C_1$-$C_6$, de préférence un groupe alkyle en $C_1$-$C_4$, qui est éventuellement monosubstitué par un radical amino, amino substitué, hydroxy, carboxy, carbamoyle, guanidino, guanidino substitué, uréido, mercapto, méthylmercapto, phényle, 4-chlorophényle, 4-fluorophényle, 4-nitrophényle, 4-méthoxyphényle, 4-hydroryphényle, phtalimido, 4-imidazolyle, 3-indolyle, 2-thiényle, 3-thiényle, 2-pyridyle, 3-pyridyle ou cyclohexyle,

le groupe amino substitué représentant un composé -NH-A- et le groupe guanidino substitué représentant un composé -NH-C(NH)-NH-A, dans lesquels A est tel que défini en $a_1$) ou $a_2$);

B représente un reste Arg, Lys, Orn, 2,4-diaminobutyroyle ou un reste L-homoarginine, le groupe amino et le groupe guanidino de la chaîne latérale pouvant étre substitués chacun par A tel que décrit en $a_1$) ou $a_2$);

C représente un composé de formule IIIa ou IIIb

G'-G'-Gly (IIIa)

G'-NH-$(CH_2)_n$-CO, (IIIb)

formules dans lesquelles les restes G' représentent, indépendamment les uns des autres, un reste de formule IV

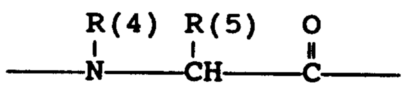

IV

dans laquelle R(4) et R(5) forment, conjointement avec les atomes qui les portent, un système cyclique hétérocyclique mono-, bi- ou tricyclique ayant de 2 à 15 atomes de carbone,

et n va de 2 à 8;

E représente le reste de la phénylalanine,
qui est éventuellement substitué en position 2, 3 ou 4 par un atome d'halogène,
le reste tyrosine, O-méthyltyrosine, 2-thiénylalanine, 2-pyridylalanine ou naphtylalanine;

F représente, indépendamment les uns des autres, le reste d'un aminoacide aliphatique ou aromatique, neutre, acide ou basique, qui peut être substitué dans la chaîne latérale, ou une liaison directe;

(D)-Tic représente le radical de formule V

A

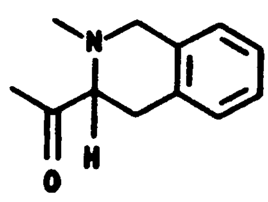

V

G est défini comme G' ou représente une liaison directe;

F' représente le reste des aminoacides basiques Arg ou Lys, sous la forme L ou D, ou une liaison directe,
le groupe guanidino ou amino de la chaîne latérale pouvant être substitué par A tel que défini en $a_1$) ou $a_2$), ou un radical -NH-$(CH_2)_n$-, dans lequel n va de 2 à 8, ou une liaison directe;

I est -OH, -$NH_2$ ou -$NHC_2H_5$;

K représente le radical -NH-$(CH_2)_x$-CO, dans lequel x va de 1 à 4, ou une liaison directe;

M est défini comme F,

et sels physiologiquement acceptables de celui-ci.

2. Peptide de formule I selon la revendication 1, dans lequel
   - B représente Arg, Orn ou Lys, le groupe guanidino ou le groupe amino de la chaîne latérale étant non substitué ou pouvant être substitué par des substituants alcanoyle en $C_1$-$C_8$, aryloyle en $C_7$-$C_{13}$, hétéroaryloyle en $C_3$-$C_9$, alkyl($C_1$-$C_8$)sulfonyle ou aryl($C_6$-$C_{12}$)sulfonyle, les radicaux aryle, hétéroaryle, aryloyle, arylsulfonyle et hétéroaryloyle pouvant éventuellement être substitués par 1, 2, 3 ou 4 radicaux identiques ou différents, comme décrit en $a_2$);
   - E représente un reste phénylalanine, 2-chlorophénylalanine, 3-chlorophénylalanine, 4-chlorophénylalanine, 2-fluorophénylalanine, 3-fluorophénylalanine, 4-fluorophénylalanine, tyrosine, O-méthyltyrosine ou $\beta$-(2-thiényl)-alanine;
   - K est une liaison directe;
   - M est une liaison directe.

3. Peptide de formule I selon la revendication 1, dans lequel
   - A représente un atome d'hydrogène, (D)- ou (L)-H-Arg, (D)-ou (L)-H-Lys ou (D)- ou (L)-H-Orn;
   - B représente Arg, Orn ou Lys,le groupe guanidino ou le groupe amino de la chalne latérale pouvant être substitué par un atome d'hydrogène ou par un groupe alcanoyle en $C_1$-$C_8$, aryloyle en $C_7$-$C_{13}$, hétéroaryloyle en $C_3$-$C_9$, alkyl($C_1$-$C_8$)sulfonyle ou aryl($C_6$-$C_{12}$)sulfonyle, les radicaux aryle, hétéroaryle, aryloyle, arylsulfonyle et hétéroaryloyle pouvant éventuellement être substitués par 1, 2, 3 ou 4 radicaux, identiques ou différents, choisis parmi des atomes d'halogène et les groupes méthyle et méthoxy;
   - C représente Pro-Pro-Gly, Hyp-Pro-Gly ou Pro-Hyp-Gly;
   - E représente Phe ou Thia;
   - F représente Ser, Hser, Lys, Leu, Val, Nle, Ile ou Thr;
   - K est une simple liaison;
   - M est une simple liaison;
   - G représente le reste d'un système cyclique hétérocyclique de formule IV, choisi parmi les restes des hétérocycles pyrrolidine (A), pipéridine (B), tétrahydroisoquinoléine (c), *cis*- ou *trans*-décahydroisoquinoléine (D), *cis*-endo-octahydroindole (E), *cis*-exo-octahydroindole (E), *trans*-octahydroindole (E), *cis*- endo-, *cis*-exo-, *trans*-octahydrocyclopentano[b]pyrrole (F) ou hydroxyproline (V);
   - F' est Arg;
   - I est OH.

4. Peptide de formule I selon la revendication 1, caractérisé en ce qu'il est choisi parmi:
   H-(D)-Arg-Arg-Pro-Hyp-Gly-Thia-Ser-(D)-Tic-Oic-Arg-OH,
   H-(D)-Arg-Arg-Pro-Pro-Gly-Thia-Ser-(D)-Tic-Oic-Arg-OH,
   H-(D)-Arg-Arg-Pro-Hyp-Gly-Phe-Ser-(D)-Tic-Oic-Arg-OH,
   H-(D)-Arg-Arg-Hyp-Pro-Gly-Phe-Ser-(D)-Tic-Oic-Arg-OH,
   H-(D)-Arg-Arg-Pro-Pro-Gly-Phe-Ser-(D)-Tic-Oic-Arg-OH.

5. Procédé pour la préparation d'un peptide de formule I selon une ou plusieurs des revendications 1 à 4, caractérisé en ce que
   a) on fait réagir un fragment à groupe carboxy C-terminal libre, ou un dérivé activé de celui-ci, avec un fragment correspondant à groupe amino N-terminal libre, ou
   b) on synthétise graduellement le peptide,

   dans le composé obtenu selon (a) ou (b), éventuellement on élimine un ou plusieurs groupes protecteurs temporairement introduits pour la protection d'autres fonctions, et éventuellement on convertit les composés de formule I ainsi obtenus en leurs sels physiologiquement acceptables.

6. Utilisation d'un peptide de formule I selon l'une des revendications 1 à 4, pour la préparation d'un médicament destiné au traitement d'états pathologiques qui sont médiés, déclenchés ou entretenus par la bradykinine ou des peptides apparentés à la bradykinine.

7. Produit pharmaceutique contenant un peptide de formule I selon une ou plusieurs des revendications 1 à 4.

**Revendications pour les Etats contractants suivants : ES, GR**

**1.** Procédé pour la préparation d'un peptide de formule I

A-B-C-E-F-K-(D)-Tic-G-M-F'-I (I)

dans laquelle

A représente

$a_1$) un atome d'hydrogène ou un groupe alkyle en $C_1$-$C_8$, alcanoyle en $C_1$-$C_8$, alcoxy-($C_1$-$C_8$)-carbonyle ou alkyl($C_1$-$C_8$)-sulfonyle,

dans chacun desquels 1, 2 ou 3 atomes d'hydrogène sont éventuellement remplacés par 1, 2 ou 3 radicaux, identiques ou différents, choisis parmi des atomes d'halogène et des groupes carboxy, amino, alkyle en $C_1$-$C_4$, alkyl($C_1$-$C_4$)-amino, hydroxy, alcoxy en $C_1$-$C_3$, dialkyl($C_1$-$C_4$)-amino, carbamoyle, sulfamoyle, alcoxy($C_1$-$C_4$)-carbonyle, aryle en $C_6$-$C_{12}$ et aryl($C_6$-$C_{12}$)-alkyle-($C_1$-$C_5$),

ou dans chacun desquels un atome d'hydrogène est éventuellement remplacé par un radical choisi parmi des radicaux cycloalkyle en $C_3$-$C_8$, alkyl($C_1$-$C_4$)-sulfonyle, alkyl($C_1$-$C_4$)-sulfinyle, aryl($C_6$-$C_{12}$)-alkyl($C_1$-$C_4$)-sulfonyle, aryl($C_6$-$C_{12}$)-alkyl($C_1$-$C_4$)-sulfinyle, aryloxy en $C_6$-$C_{12}$, hétéroaryle en $C_3$-$C_9$ et hétéroaryloxy en $C_3$-$C_9$,

et 1 ou 2 atomes d'hydrogène sont remplacés par 1 ou 2 radicaux, identiques ou différents, choisis parmi des atomes d'halogène et des groupes carboxy, amino, alkyl-($C_1$-$C_4$)amino, hydroxy, alcoxy en $C_1$-$C_4$, dialkyl(-$C_1$-$C_4$)-amino, carbamoyle, sulfamoyle, alkyloxy($C_1$-$C_4$)-carbonyle, aryle en $C_6$-$C_{12}$ et aryl($C_6$-$C_{12}$)-alkyle($C_1$-$C_5$),

$a_2$) un groupe cycloalkyle en $C_3$-$C_8$ ou carbamoyle,

qui peut éventuellement être substitué à l'azote par un radical alkyle en $C_1$-$C_6$ ou aryle en $C_6$-$C_{12}$,

un groupe aryle en $C_6$-$C_{12}$, aryloyle en $C_7$-$C_{18}$, aryl-($C_6$-$C_{12}$)sulfonyle ou hétéroaryle en $C_3$-$C_9$ ou hétéroaryloyle en $C_3$-$C_9$,

dans les restes définis en $a_1$) et $a_2$), les radicaux hétéroaryle, aryloyle, arylsulfonyle et hétéroaryloyle étant éventuellement substitués chacun par 1, 2, 3 ou 4 radicaux différents choisis parmi des atomes d'halogène et des groupes carboxy, amino, nitro, alkyl($C_1$-$C_4$)-amino, hydroxy, alkyle en $C_1$-$C_4$, alcoxy en $C_1$-$C_4$, cyano, dialkyl($C_1$-$C_4$)-amino, carbamoyle, sulfamoyle et alcoxy($C_1$-$C_4$)-carbonyle, ou

$a_3$) un radical de formule II

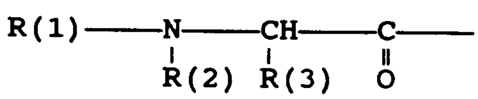

(II)

dans laquelle

R(1) est défini comme A en $a_1$) ou $a_2$),

R(2) représente un atome d'hydrogène ou le groupe méthyle,

R(3) représente un atome d'hydrogène ou un groupe alkyle en $C_1$-$C_6$, de préférence un groupe alkyle en $C_1$-$C_4$, qui est éventuellement monosubstitué par un radical amino, amino substitué, hydroxy, carboxy, carbamoyle, guanidino, guanidino substitué, uréido, mercapto, méthylmercapto, phényle, 4-chlorophényle, 4-fluorophényle, 4-nitrophényle, 4-méthoxyphényle, 4-hydroxyphényle, phtalimido, 4-imidazolyle, 3-indolyle, 2-thiényle, 3-thiényle, 2-pyridyle, 3-pyridyle ou cyclohexyle,

le groupe amino substitué représentant un composé -NH-A- et le groupe guanidino substitué représentant un composé -NH-C(NH)-NH-A, dans lesquels A est tel que défini en $a_1$) ou $a_2$);

B représente un reste Arg, Lys, Orn, 2,4-diaminobutyroyle ou un reste L-homoarginine, le groupe amino et le groupe guanidino de la chaine latérale pouvant être substitués chacun par A tel que décrit en $a_1$) ou $a_2$);

C représente un composé de formule IIIa ou IIIb

G'-G'-Gly (IIIa)

G'-NH-$(CH_2)_n$-CO, (IIIb)

formules dans lesquelles les restes G' représentent, indépendamment les uns des autres, un reste de formule IV

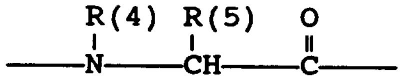

IV

dans laquelle R(4) et R(5) forment, conjointement avec les atomes qui les portent, un système cyclique hétérocyclique mono-, bi- ou tricyclique ayant de 2 à 15 atomes de carbone,
et n va de 2 à 8;

E représente le reste de la phénylalanine,
qui est éventuellement substitué en position 2, 3 ou 4 par un atome d'halogène,
le reste tyrosine, O-méthyltyrosine, 2-thiénylalanine, 2-pyridylalanine ou naphtylalanine;

F représente, indépendamment les uns des autres, le reste d'un aminoacide aliphatique ou aromatique, neutre, acide ou basique, qui peut être substitué dans la chaine latérale, ou une liaison directe;

(D)-Tic représente le radical de formule V

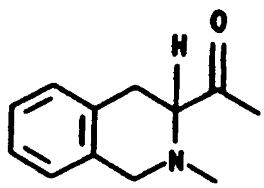

V

V

G est défini comme G' ou représente une liaison directe;

F' représente le reste des aminoacides basiques Arg ou Lys, sous la forme L ou D, ou une liaison directe,
le groupe guanidino ou amino de la chaîne latérale pouvant être substitué par A tel que défini en $a_1$) ou $a_2$), ou un radical -NH-$(CH_2)_n$-, dans lequel n va de 2 à 8, ou une liaison directe;

I est -OH, $-NH_2$ ou $-NHC_2H_5$;

K représente le radical -NH-$(CH_2)_x$-CO, dans lequel x va de 1 à 4, ou une liaison directe;

M est défini comme F,

et de ses sels physiologiquement acceptables, caractérisé en ce que

a) on fait réagir un fragment à groupe carboxy C-terminal libre, ou un dérivé activé de celui-ci, avec un fragment correspondant à groupe amino N-terminal libre, ou

b) on synthétise graduellement le peptide,

dans le composé obtenu selon (a) ou (b), éventuellement on élimine un ou plusieurs groupes protecteurs temporairement introduits pour la protection d'autres fonctions, et éventuellement on convertit les composés de formule I ainsi obtenus en leurs sels physiologiquement acceptables.

**2.** Procédé selon la revendication 1, pour la préparation d'un peptide de formule I, dans lequel

B représente Arg, Orn ou Lys,
le groupe guanidino ou le groupe amino de la chaine latérale étant non substitué ou pouvant être substitué par des substituants alcanoyle en $C_1$-$C_8$, aryloyle en $C_7$-$C_{13}$, hétéroaryloyle en $C_3$-$C_9$, alkyl($C_1$-$C_8$)sulfonyle ou aryl($C_6$-$C_{12}$)sulfonyle, les radicaux aryle, hétéroaryle, aryloyle, arylsulfonyle et hétéroaryloyle pouvant éventuellement être substitués par 1, 2, 3 ou 4 radicaux identiques ou différents, comme décrit en $a_2$);

E représente un reste phénylalanine, 2-chlorophénylalanine, 3-chlorophénylalanine, 4-chlorophénylalanine, 2-fluorophénylalanine, 3-fluorophénylalanine, 4-fluorophénylalanine, tyrosine, O-méthyltyrosine ou $\beta$-(2-thiényl)-alanine;

K est une liaison directe;

M est une liaison directe.

**3.** Procédé selon la revendication 1, pour la préparation d'un peptide de formule I, dans lequel
A représente un atome d'hydrogène, (D)- ou (L)-H-Arg, (D)-ou (L)-H-Lys ou (D)- ou (L)-H-Orn;
B représente Arg, Orn ou Lys,le groupe guanidino ou le groupe amino de la chaîne latérale pouvant être substitué par un atome d'hydrogène ou par un groupe alcanoyle en $C_1$-$C_8$, aryloyle en $C_7$-$C_{13}$, hétéroaryloyle en $C_3$-$C_9$, alkyl($C_1$-$C_8$)sulfonyle ou aryl($C_6$-$C_{12}$)sulfonyle, les radicaux aryle, hétéroaryle, aryloyle, arylsulfonyle et hétéroaryloyle pouvant éventuellement être substitués par 1, 2, 3 ou 4 radicaux, identiques ou différents, choisis parmi des atomes d'halogène et les groupes méthyle et méthoxy;
C représente Pro-Pro-Gly, Hyp-Pro-Gly ou Pro-Hyp-Gly;
E représente Phe ou Thia;
F représente Ser, Hser, Lys, Leu, Val, Nle, Ile ou Thr;
K est une simple liaison;
M est une simple liaison;
G représente le reste d'un système cyclique hétérocyclique de formule IV, choisi parmi les restes des hétérocycles pyrrolidine (A), pipéridine (B), tétrahydroisoquinoléine (c), *cis*- ou *trans*-décahydroisoquinoléine (D), *cis*-endo-octahydroindole (E), *cis*-exo-octahydroindole (E), *trans*-octahydroindole (E), *cis*- endo-, *cis*-exo-, *trans*-octahydrocyclopentano[b]pyrrole (F) ou hydroxyproline (V);
F' est Arg;
I est OH.

**4.** Procédé selon la revendication 1, pour la préparation d'un peptide de formule I, dans lequel on prépare un peptide choisi parmi:
H-(D)-Arg-Arg-Pro-Hyp-Gly-Thia-Ser-(D)-Tic-Oic-Arg-OH,
H-(D)-Arg-Arg-Pro-Pro-Gly-Thia-Ser-(D)-Tic-Oic-Arg-OH,
H-(D)-Arg-Arg-Pro-Hyp-Gly-Phe-Ser-(D)-Tic-Oic-Arg-OH,
H-(D)-Arg-Arg-Hyp-Pro-Gly-Phe-Ser-(D)-Tic-Oic-Arg-OH,
H-(D)-Arg-Arg-Pro-Pro-Gly-Phe-Ser-(D)-Tic-Oic-Arg-OH.

**5.** Utilisation d'un peptide de formule I, préparé selon l'une des revendications 1 à 4, pour la préparation d'un médicament destiné au traitement d'états pathologiques qui sont médiés, déclenchés ou entretenus par la bradykinine ou des peptides apparentés à la bradykinine.